(19) Europäisches Patentamt
European Patent Office
Office européen des brevets

(11) **EP 4 205 778 B1**

(12) **EUROPEAN PATENT SPECIFICATION**

(45) Date of publication and mention
of the grant of the patent:
**03.12.2025  Bulletin 2025/49**

(21) Application number: **21306959.4**

(22) Date of filing: **30.12.2021**

(51) International Patent Classification (IPC):
***A61M 1/36*** (2006.01)

(52) Cooperative Patent Classification (CPC):
**A61M 1/3656;** A61M 1/3639; A61M 2205/18

(54) **APPARATUS FOR EXTRACORPOREAL BLOOD TREATMENT**

VORRICHTUNG ZUR EXTRAKORPORALEN BLUTBEHANDLUNG

APPAREIL POUR TRAITEMENT SANGUIN EXTRACORPOREL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.07.2023  Bulletin 2023/27**

(73) Proprietor: **Gambro Lundia AB
226 43 Lund (SE)**

(72) Inventor: **POUCHOULIN, Dominique
01390 Tramoyes (FR)**

(74) Representative: **Ponzellini, Gian-Marco
PGA S.p.A., Milano
Succursale di Lugano
Via Castagnola 21C
6900 Lugano (CH)**

(56) References cited:
**US-A1- 2016 354 531     US-A1- 2018 126 062**

**Description**

**Technical Field**

The present invention relates to an apparatus for extracorporeal blood treatment capable of detecting disconnection events during treatment. For instance, the present invention is applicable in the context of medical apparatuses and methods for continuous renal replacement therapies (CRRT) or other therapies in Intensive Care Units (ICU), like Extra-Corporeal CO2 Removal (ECCO2R), Hemo-Perfusion (HP) or Therapeutic Plasma Exchange (TPE).

**Background of the Invention**

CRRT systems are configured for delivering treatments designed for patients versing in acute states of illness and who have temporarily lost their kidney function in its entirety. CRRT monitors should be able to deliver various therapies, e.g.: ultrafiltration (UF), continuous veno venous hemofiltration (CCVH), continuous veno venous hemodiafiltration (CVVHDF), continuous veno venous hemodialysis (CCVHD).

Within the context of CRRT, in which the vascular access is typically performed via a catheter, the disconnection of the line or lines from the catheter may occur and is critical for the risk of blood loss or air embolism. Indeed, return line disconnection events with no detection from the system may lead to patient death by exsanguination driven by the blood pump. Known systems for the detection of return line disconnection are based on monitoring the return pressure and on the assessment of at least one of the following conditions:

- decrease of the return pressure from a reference value (operating point) by more than X mmHg, wherein the value of X can be automatically set by the system and/or be adjusted by an operator;
- return pressure below a fixed threshold value Y, which could be again automatically set by the system or be operator adjustable.

Experience indicates that above design is not reliable, since most alarm occurrences do not match with disconnection events and some actual disconnection events are not detected.

Document WO2018053461A1 is also known, which discloses a method and a system for detecting a condition indicative of a dislodged needle in a hemodialysis procedure. A venous return pressure for a patient undergoing dialysis is measured. The venous return pressure is analyzed via a controller and an intravascular blood pressure in proximity to a location of needle insertion into the patient is derived. A lower limit is calculated as a function of the intravascular blood pressure via the controller. An average of the venous return pressure is calculated via the controller during a predetermined time window. The average is compared to the lower limit via the controller and, if the average is within a range of the lower limit, the controller determines that a condition indicative of a dislodged needle is present. Public document US 2016/0354531 A1 discloses a method and a device for monitoring a vascular access during an extracorporeal blood treatment. The method and the device are based on the monitoring of the difference between the venous pressure measured by a venous pressure sensor and the arterial pressure measured by an arterial pressure sensor in the extracorporeal blood circuit.

Public document US 2018/0126062 A1 discloses a monitoring system that performs a method for detecting a disruption of a fluid connection between a first fluid containing system, like an extracorporeal circuit for blood processing, and a second fluid containing system, like a human subject. The monitoring system generates a monitoring signal which is representative of a fluid pressure in respect of the first fluid containing system and which is responsive to the disruption of the fluid connection, and a tracking signal which corresponds to and is more smoothed over time than the monitoring signal.

**Glossary**

The following terms/parameters are consistently used throughout the equations provided in the following description and in the appended claims.

| Terms/Parameters | |
|---|---|
| $P_{H\_patient}$ | hydrostatic pressure difference |
| $P_{Hret\_patient}$ | hydrostatic pressure difference return line |
| $P_{Hwith\_patient}$ | hydrostatic pressure difference withdrawal line |
| $\Delta P_{line}$ | section pressure drop |

(continued)

| Terms/Parameters | |
|---|---|
| $\Delta P_{ret\_line}$ | section pressure drop return line |
| $\Delta P_{with\_line}$ | section pressure drop withdrawal line |
| $P_{disc}$ | disconnection pressure |
| $P_{ret\_disc}$ | return disconnection pressure |
| $P_{with\_disc}$ | withdrawal disconnection pressure |
| $P_{thresh}$ | pressure alarm threshold |
| $P_{ret\_thresh}$ | pressure alarm threshold return line |
| $P_{with\_thresh}$ | pressure alarm threshold withdrawal line |
| $P_{with\_thresh}$ | measured pressure |
| $P_{ret}$ | measured pressure return line |
| $P_{with}$ | measured pressure withdrawal line |
| $P_{offset}$ | offset pressure |
| $P_{offset\_ret}$ | offset pressure return line |
| $P_{offset\_with}$ | offset pressure withdrawal line |
| $P_{Qb0}$ | static pressure |
| $P_{ret\_Qb0}$ | static pressure return line |
| $P_{with\_Qb0}$ | static pressure withdrawal line |
| $\Delta P_{cath}$ | pressure drop catheter |
| $\Delta P_{ret\_cath}$ | pressure drop catheter return line |
| $\Delta P_{with\_cath}$ | pressure drop catheter withdrawal line |
| $\Delta P_{safety}$ | safety margin |
| $\Delta P_{ret\_safety}$ | safety margin return line |
| $\Delta P_{with\_safety}$ | safety margin withdrawal line |
| $P_{venous}$ | patient central venous pressure |
| $Qb$ | blood flow rate |
| $Qb0$ | zero blood flow rate |
| $H$ | difference in height |
| $H_{ret}$ | difference in height return pressure sensor |
| $H_{with}$ | difference in height withdrawal pressure sensor |
| $k_{line}$ | section pressure drop coefficient |
| $k_{ret\_line}$ | section pressure drop coefficient return line |
| $k_{with\_line}$ | section pressure drop coefficient withdrawal line |
| $L$ | length of the section |
| $d$ | internal diameter of the section |
| $L_{with}$ | length of the section return line |
| $d_{with}$ | internal diameter of the section return line |
| $L_{ret}$ | length of the section withdrawal line |
| $d_{ret}$ | internal diameter of the section withdrawal line |
| $\mu$ | blood viscosity |

## Summary

In this situation, it is a general object of the present invention to offer a technical solution capable overcoming one or more of the above drawbacks.

It is an object of the present invention to improve the reliability of detection of events of disconnection of the blood circuit (i.e. return line and/or withdrawal line) from the patient during extracorporeal blood treatments and thus improving safety of the patient.

More in detail, it is an aim of the present invention to provide an apparatus and a method allowing to detect the actual disconnection events and to reduce occurrence of false alarms. It is also an aim of the present invention to manage clotting events which may develop along the therapy.

It is also an aim of the present invention to provide a reliable detection system which does not have a negative impact on costs of the apparatus and/or of the treatments.

It is also an aim of the present invention to provide a reliable detection system which may be easily implemented in current extracorporeal blood treatment apparatuses and which does not require improvements of the hardware of these apparatuses.

At least one of the above objects is substantially reached by an apparatus for extracorporeal blood treatment according to one or more of the appended claims.

## Description of the drawings

Aspects of the invention are shown in the attached drawings, which are provided by way of non-limiting examples, wherein:

Figure 1 shows a schematic representation of an extracorporeal blood treatment apparatus;
Figure 2 shows a schematic view in side elevation of the extracorporeal blood treatment apparatus of Figure 1;
Figure 3 shows an element of the extracorporeal blood treatment apparatus of Figures 1 and 2;
Figure 4 is a flowchart showing a method of detecting disconnection events in an apparatus for extracorporeal blood treatment according to aspects of the invention;
Figures 5 and 6 represent a flowchart showing part of another method of detecting disconnection events in an apparatus for extracorporeal blood treatment according to aspects of the invention;
Figure 7 is a flowchart showing another part of the method of figures 5 and 6.

## Detailed description

### Extracorporeal blood treatment apparatus

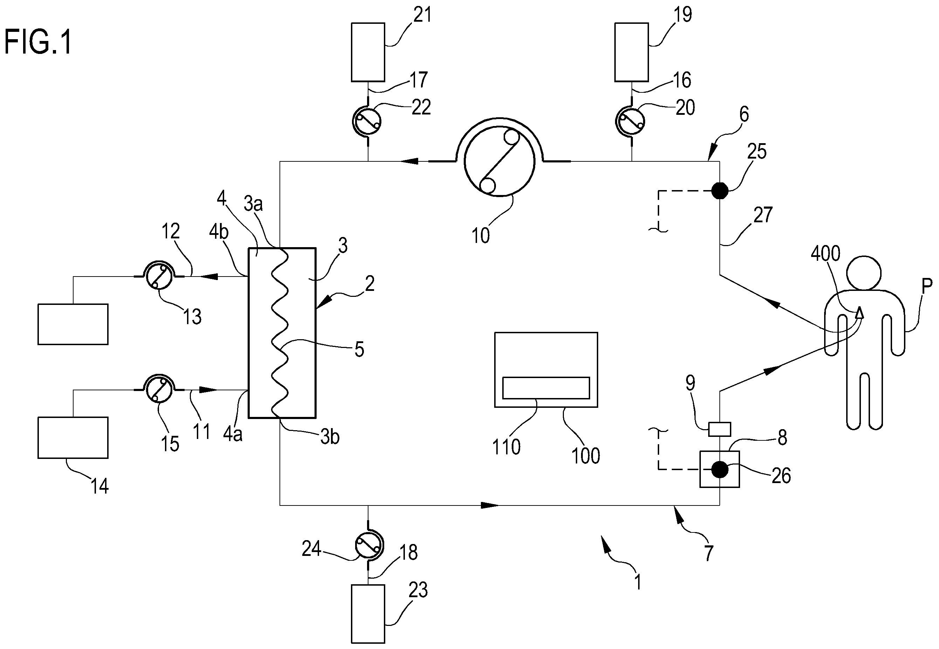

An apparatus 1 for extracorporeal blood treatment is schematically represented in Figure 1. The apparatus 1 is a continuous renal replacement therapy (CRRT) apparatus for intensive care treatments, for instance configured to deliver various therapies, like CCVH, CVVHDF, CVVHD, SCUF.

The apparatus 1 comprises a treatment or filtration unit 2 having a primary chamber 3 and a secondary chamber 4 separated by a semi-permeable membrane 5. Depending upon the treatment, the semi-permeable membrane 5 of the filtration unit 2 may be selected to have different properties and performances.

A blood circuit is coupled to the primary chamber 3 of the filtration unit 2. The blood circuit comprises a blood withdrawal line 6 connected to an inlet 3a of the primary chamber 3, a blood return line 7 connected to an outlet 3b of the primary chamber 3. The blood withdrawal line 6 and blood return line 7 are configured for connection to a cardiovascular system of a patient "P".

In use, the blood withdrawal line 6 and the blood return line 7 are connected to a vascular access device 400 which is then placed in fluid communication with the patient "P" vascular system, such that blood may be withdrawn through the blood withdrawal line 6, flown through the primary chamber 3 and then returned to the patient's vascular system through the blood return line 7.

An air detector, not shown, and an air separator, such as a deaeration chamber 8, may be present on the blood return line 7. Moreover, a monitor valve 9 may be present on the blood return line 7, downstream the deaeration chamber 8.

The blood flow through the blood circuit is controlled by a blood pump 10, for instance a peristaltic blood pump, acting either on the blood withdrawal line 6 or on the blood return line 7. The embodiment of Figure 1 shows the blood pump 10 coupled to a pump section of the blood withdrawal line 6.

A dialysis circuit is connected to the secondary chamber 4 of the filtration unit 2 and comprises a dialysis line 11 connected to an inlet 4a of the secondary chamber 4 and an effluent line 12 connected to an outlet 4b of the secondary chamber 4 and to a drain, not shown.

An effluent pump 13 is located on the effluent line 12 and is able to recall fluid from the second chamber 4. The dialysis line 11 is connected to a source 14, e.g. a bag or a preparation device, of fresh dialysis fluid and a dialysis pump 15 is located on the dialysis line 11 and is able to pump fluid to the second chamber 4.

The apparatus 1 further comprises an infusion circuit comprising at least one infusion line. The infusion circuit shown in the embodiment of Figure 1 comprises a pre-blood pump line 16, a pre-infusion line 17 and a post-infusion line 18.

The pre-blood pump line 16 is connected to the blood withdrawal line 6 upstream of the blood pump 10 and to a first source 19 of infusion fluid, e.g. a bag. A pre-blood pump 20 is located on the pre-blood pump line 16 and is able to pump fluid from the first source 19 to the blood circuit.

The pre-infusion line 17 is connected to the blood withdrawal line 6 downstream of the blood pump 10 and upstream of the filtration unit 2 and to a second source 21 of infusion fluid, e.g. a bag. A pre-infusion pump 22 is located on the pre-infusion line 17 and is able to pump fluid from the second source 21 to the blood circuit.

The post-infusion line 18 is connected to the blood return line 7 downstream of the filtration unit 2 and to a third source 23 of infusion fluid, e.g. a bag. A post-infusion pump 24 is located on the post-infusion line 18 and is able to pump fluid from the third source 23 to the blood circuit.

The apparatus 1 may also comprise one or more auxiliary line/s, not shown, connected to the blood circuit and to a source of at least one compensation substance or of an anticoagulant, such as potassium or bicarbonate, and a pump or syringe configured to deliver a flow rate of the compensation substance, such as potassium or bicarbonate, etc..

A withdrawal pressure sensor 25 is configured to detect a pressure at a measurement location in the blood withdrawal line 6. A return pressure sensor 26 is configured to detect a pressure at a measurement location in the blood return line 7. The withdrawal pressure sensor 25 and the return pressure sensor 26 may comprise pressure pods in the blood withdrawal line 6 and blood return line 7. The return pressure sensor 26 may be operatively coupled to the deaeration chamber 8, as shown in Figure 1.

A control unit 100 is connected and controls the blood pump 10, the dialysis pump 15, the effluent pump 13, the pre-blood pump 20, the pre-infusion pump 22 and the post-infusion pump 24 to regulate a blood flow rate "$Q_b$" in the blood circuit, a dialysis flow rate crossing the dialysis line 11, an effluent flow rate crossing the effluent line 12, an infusion flow rate crossing the pre-blood pump line 16, an infusion flow rate crossing the pre-infusion line 17, an infusion flow rate crossing the post-infusion line 18. Through the control of the dialysis flow rate crossing the dialysis line 11 and/or of the effluent flow rate crossing the effluent line 12, the control unit 100 is also configured to control/regulate a filtration flow rate (through the control of the dialysis pump 15 and the effluent pump 13) in the filtration unit 2 and/or a patient fluid removal rate (also through the control of the pre-blood pump 20, the pre-infusion pump 22 and the post-infusion pump 24).

The control unit 100 is also connected to the withdrawal pressure sensor 25 and to the return pressure sensor 26 to receive signals correlated to pressure values from these sensors 25, 26. The withdrawal pressure sensor 25 and the return pressure sensor 26 provide to the control unit 100 signals correlated to pressure in the extracorporeal blood circuit.

The control unit 100 may be an electronic control unit comprising at least a CPU, a memory and input/output devices. The control unit 100 comprises or is connected to an interface 110 configured to display data and/or allow a user to input data. For instance, the interface comprises a display, e.g. a touch screen, and/or buttons or a keyboard.

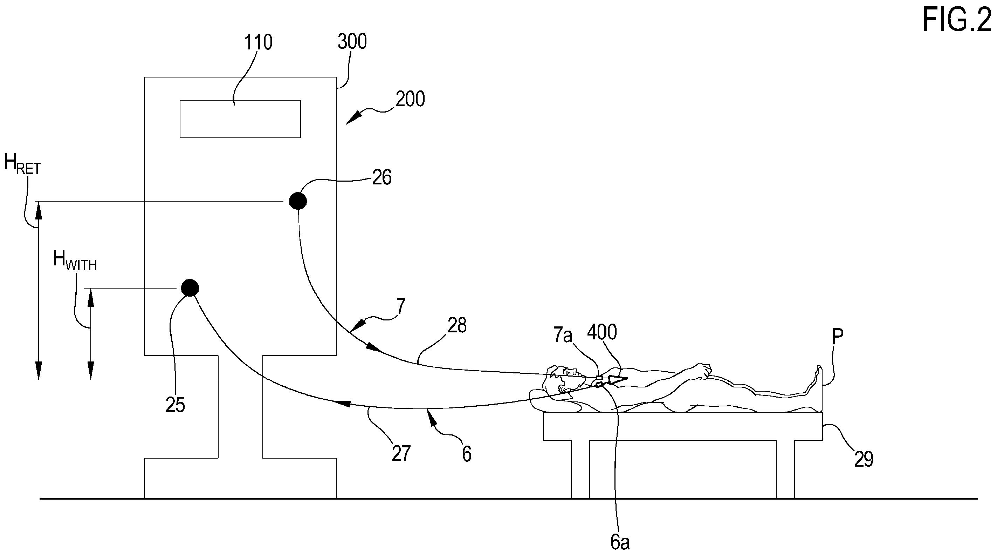

The apparatus 1 may comprise a treatment machine 200 and an integrated disposable set configured to be coupled to the treatment machine 200. The outline of the treatment machine 200 is represented schematically in Figure 2.

The treatment machine 200 comprises the cited blood pump 10, effluent pump 13, dialysis pump 15, pre-blood pump 20, pre-infusion pump 22, post-infusion pump 24, control unit 100 with the interface 110, flow rate sensors.

The treatment machine 200 may comprise also the withdrawal pressure sensor 25 and the return pressure sensor 26 or the withdrawal pressure sensor 25 and the return pressure sensor 25 may be part of the integrated disposable set. The treatment machine 200 comprises also all the other elements and/or devices configured to receive and hold parts of the integrated disposable set.

The integrated disposable set comprises the treatment or filtration unit 2, the blood circuit, the effluent line 12, the dialysis line 11, the infusion lines 16, 17, 18, which are grouped together.

When the integrated disposable set is mounted on the treatment machine 200, the withdrawal pressure sensor 25 and the return pressure sensor 26 or the measurement locations of the withdrawal pressure sensor 25 and return pressure sensor 26 are in fixed positions on a frame 300 of the treatment machine 200 and at predefined heights above the ground.

A section 27 of the blood withdrawal line 6 develops from the withdrawal pressure sensor 25 on the treatment machine 200 to the vascular access device 400 and to the patient P undergoing treatment. A section 28 of the blood return line 7 develops from the return pressure sensor 26 on the treatment machine 200 to the vascular access device 400 and to the patient P.

As shown in Figure 2, the patient P undergoing treatment is lying on a bed 29 and the vascular access device 400 is placed at a height which may be different from a height of the withdrawal pressure sensor 25 and of the return pressure sensor 26 and may vary with respect to both the heights of the withdrawal pressure sensor 25 and return pressure sensor 26, since the bed height is typically adjustable.

**Vascular access device**

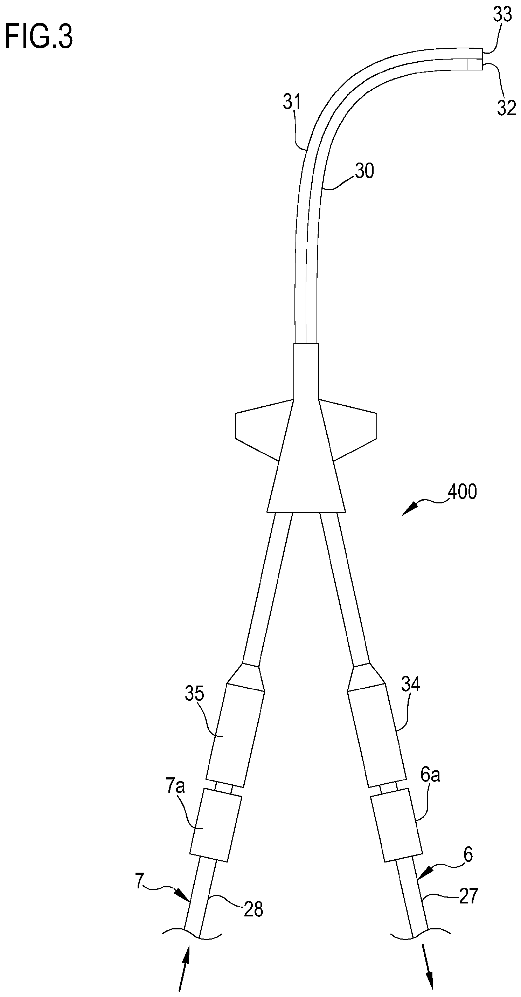

The vascular access device 400 shown in Figure 3 is central double lumen venous catheter (CVC) which is configured to be placed in a large central vein of the patient P, for instance in the neck (internal jugular vein).

The vascular access device 400 comprises a withdrawal section 30 delimiting a withdrawal lumen and a return section 31 delimiting a return lumen. Distal portions of the withdrawal section 30 and return section 31 are provided respectively with a distal tip 32 of the withdrawal section 30 and a distal tip 33 of the return section. The distal portions of the withdrawal section 30 and return section 31 are paired and configured to be placed inside the large central vein. Proximal portions of the withdrawal section 30 and return section 31 are split and configured to remain outside the patient body.

The proximal portions are provided respectively with a withdrawal port 34 and a return port 35. The withdrawal port 34 is connected or configured to be connected to a connector 6a of the blood withdrawal line 6 and the return port 35 is connected or configured to be connected to a connector 7a of the blood return line 7, as shown in Figure 3, and allow to connect the extracorporeal blood circuit to the vascular system of the patient P.

**Detection of disconnection events**

According to a method of detecting disconnection events in an apparatus for extracorporeal blood treatment, the control unit 100 is configured and/or programmed for detecting a disconnection between the withdrawal port 34 and the connector 6a of the blood withdrawal line 6 and/or between the return port 35 and the connector 7a of the blood return line 7 while the patient P is undergoing an extracorporeal blood treatment. When a disconnection event is detected, the control unit 100 is configured and/or programmed for sending an alarm or a warning signal and/or stopping the extracorporeal blood treatment.

**Disconnection of return line**

During treatment (i.e. when the blood pump 10 is running), the return pressure $P_{ret}$ at the measurement location of the return pressure sensor 26 is given by the following equation:

$$Eq.1) \qquad P_{ret} = P_{offset\_ret} + \Delta P_{ret\_cath} + \Delta P_{ret\_line}$$

wherein

- $P_{offset\_ret} = P_{ret\_Qb0}$ is the pressure when there is no blood flow rate (Qb = 0);
- $\Delta P_{ret\_cath}$ is the pressure drop in the return section 31 of the vascular access device 400;
- $\Delta P_{ret\_line}$ is the pressure drop in the section 28 of the blood return line 7 from the connector 7a of the blood return line 7 to the return pressure sensor 26.

The return pressure at the measurement location $P_{ret\_Qb0}$ when there is no blood flow rate (Qb = 0) is given by the following equation:

$$Eq.2) \qquad P_{offset\_ret} = P_{ret\_Qb0} = P_{venous} + P_{Hret\_patient}$$

wherein

- $P_{venous}$ is a patient central venous pressure;
- $P_{Hret\_patient}$ is a hydrostatic pressure difference in the blood return line 7 due to a difference in height $H_{ret}$ between the vascular access device 400 and the return pressure sensor 26.

Indeed, in the case of zero blood flow rate, there is no pressure drop along the blood circuit and only hydrostatic pressure as well as patient central venous pressure define the offset pressure.

Therefore equation 1 becomes:

$$Eq.1') \qquad P_{ret} = P_{venous} + P_{Hret\_patient} + \Delta P_{ret\_cath} + \Delta P_{ret\_line}$$

In case of disconnection between the connector 7a of the blood return line 7 and the return port 35 of the vascular access device 400, the return pressure at the measurement location (the return disconnection pressure $P_{ret\_disc}$) is given by the following equation:

$$\text{Eq.3)} \qquad P_{ret\_disc} = P_{Hret\_patient} + \Delta P_{ret\_line}$$

because, if the connector 7a is disconnected from the return port 35, the return pressure at the measurement location is not affected by the patient central venous pressure $P_{venous}$ and by the pressure drop $\Delta P_{ret\_cath}$ in the return section of the vascular access device 400.

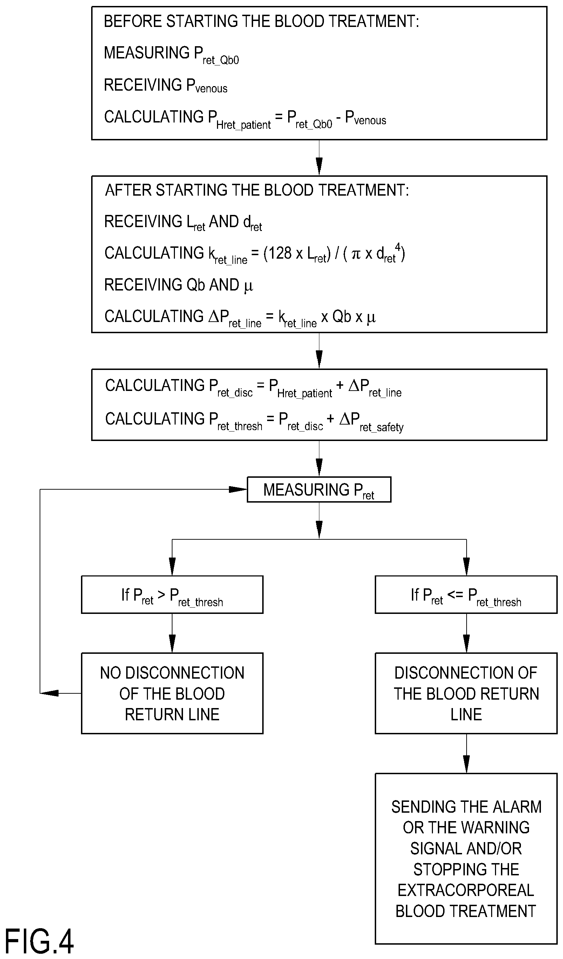

**Example 1 (Figure 4)**

In view of equations 1), 1'), 2) and 3) above, in order to detect the disconnection of the blood return line 7, before starting the blood treatment and after the patient has been connected, while the blood pump 10, the effluent pump 13, the dialysis pump 15, the pre-blood pump 20, the pre-infusion pump 22 and the post-infusion pump 24 are stopped and the blood flow rate is zero (Qb0), the control unit 100 is configured for performing the following steps:

- measuring the return pressure $P_{ret\_Qb0}$ at the measurement location;
- receiving the patient central venous pressure $P_{venous}$; and
- calculating the hydrostatic pressure difference $P_{Hret\_patient}$ in
- the blood return line 7 starting from equation 2) as:

$$\text{Eq.4)} \qquad P_{Hret\_patient} = P_{ret\_Qb0} - P_{venous}$$

In equation 4), the central venous pressure $P_{venous}$ may be set as a default value, for instance between +6 mmHg (799.93 Pa) and +12 mmHg (1599.87 Pa), e.g. of +10 mmHg (1333.22 Pa), because typical values are reported in the 6-12 mmHg (799.93 - 1599.87 Pa) range. The central venous pressure $P_{venous}$ may also be measured and entered into the control unit 100 through a query to the operator/medical staff.

After starting the blood treatment, the control unit 100 is configured for performing the following steps:

- receiving the blood flow rate Qb (e.g. 200 ml/min);
- receiving a blood viscosity $\mu$ (e.g. 3 mPa s);
- receiving a length $L_{ret}$ (e.g. 2.2 m) and an internal diameter $d_{ret}$ (e.g. 4.5 mm) of the section 28 of the blood return line 7;
- calculating a section pressure drop coefficient of the blood return line $k_{ret\_line}$ through the following equation:

$$\text{Eq.5)} \qquad k_{ret\_line} = (128 \times L_{ret})/(\pi \times d_{ret}^4)$$

(e.g. 0.0277 mmHg/ (ml/min) / (mPa s) or 3.693 Pa/(ml/min)/(mPa s))
- calculating the pressure drop $\Delta P_{ret\_line}$ in the section 28 of the blood return line 7 through the following equation:

$$\text{Eq.6)} \qquad \Delta P_{ret\_line} = k_{ret\_line} \times Qb \times \mu$$

(e.g. 17 mmHg or 2266 Pa)
- calculating the return disconnection pressure $P_{ret\_disc}$ through equation 3 (e.g. 2 mmHg (267 Pa) assuming $P_{venous}$ = 10 mmHg (1333.22 Pa) and $P_{ret\_Qb0}$ = - 5 mmHg (667 Pa));
- setting a pressure alarm threshold $P_{ret\_thresh}$ of the blood return line 7 as:

$$\text{Eq.7)} \qquad P_{ret\_thresh} = P_{ret\_disc} + \Delta P_{ret\_safety}$$

(safety margin) (e.g. 12 mmHg (1599.87 Pa) assuming a safety margin of 10 mmHg (1333.22 Pa))

Once the pressure alarm threshold $P_{ret\_thresh}$ has been calculated, the control unit 100 is configured for performing the following steps:

- receiving the measured return pressure $P_{ret}$ and comparing with the pressure alarm threshold $P_{ret\_thresh}$ of the blood return line 7 (with a second or a few seconds frequency);
- sending the alarm or the warning signal and/or stopping the extracorporeal blood treatment if the measured return pressure $P_{ret}$ is equal to or lower than the pressure alarm threshold $P_{ret\_thresh}$.

If $P_{ret} > P_{ret\_thresh}$ no disconnection of the blood return line 7; If $P_{ret} <= P_{ret\_thresh}$ disconnection of the blood return line 7.

In this Example 1, the section pressure drop coefficient $k_{ret\_line}$ of the blood return line 7 is a constant along the extracorporeal blood treatment. If the blood flow rate Qb and the blood viscosity $\mu$ remain unchanged, also the pressure drop $\Delta P_{ret\_line}$ calculated through equation 6) remains unchanged even if the calculation is repeated.

The hydrostatic pressure difference $P_{Hret\_patient}$ may change due to changes of bed height and/or of patient position. Therefore, the calculation of the hydrostatic pressure difference $P_{Hret\_patient}$, which is performed before starting the blood treatment through equation 4), is repeated every 6 to 12 hours along the treatment and each time a change in bed height and/or patient position is reported.

In order to repeat the measurements of the return pressure $P_{ret\_Qb0}$ and the calculation of the hydrostatic pressure difference $P_{Hret\_patient}$, the pumps (the blood pump 10, the effluent pump 13, the dialysis pump 15, the pre-blood pump 20, the pre-infusion pump 22 and the post-infusion pump 24) are stopped and the static pressure $P_{Qb0}$ is measured when becoming stable.

Then, the return disconnection pressure $P_{ret\_disc}$ and the pressure alarm threshold $P_{ret\_thresh}$ are updated accordingly.

The safety margin $\Delta P_{ret\_safety}$ may be e.g. 10 mmHg (1333.22 Pa) or may be also set to zero, such that the pressure alarm threshold $P_{ret\_thresh}$ of the blood return line 7 is equal to the return disconnection pressure $P_{ret\_disc}$.

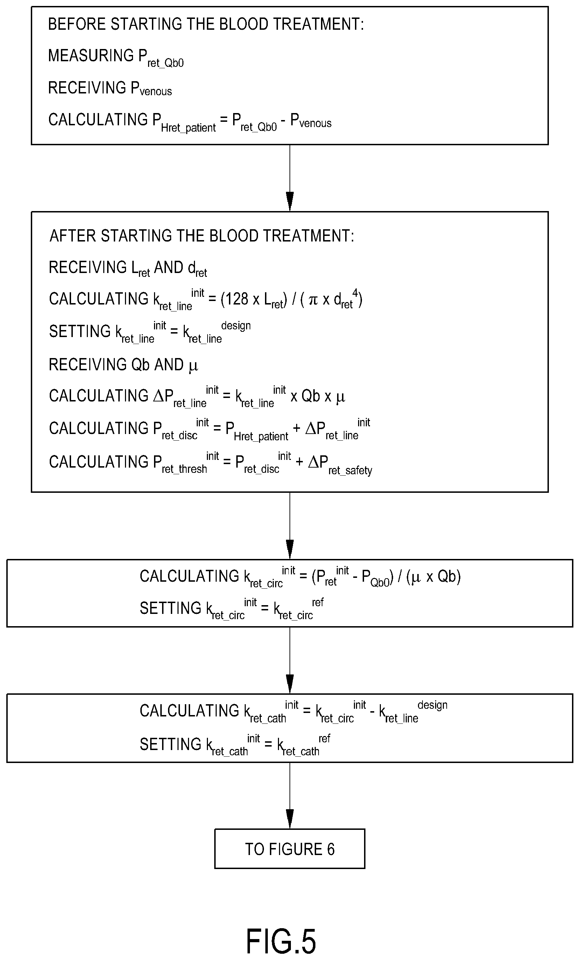

## Example 2 (Figures 5, 6 and 7)

According to Example 2, the section pressure drop coefficient $k_{ret\_line}$ of the blood return line 7 and consequently also the pressure drop $\Delta P_{ret\_line}$ in the section 28 of the blood return line 7, the return disconnection pressure $P_{ret\_disc}$ and the pressure alarm threshold $P_{ret\_thresh}$ are continuously updated during the extracorporeal blood treatment (e.g. every 1 to 10 minutes). This example 2 allows to take into account the presence of clotting in the blood return line 7, since the catheter pressure drop coefficient $k_{ret\_cath}$ and the section pressure drop coefficient $k_{ret\_line}$ of the blood return line 7 may change along treatment further to clotting problems.

At the start of the extracorporeal blood treatment, the control unit 100 is configured for performing the following steps:

- receiving the blood flow rate Qb (e.g. 150 ml/min);
- receiving the blood viscosity $\mu$ (e.g. 3.0 mPa s);
- receiving the length $L_{ret}$ (e.g. 246 cm) and the internal diameter $d_{ret}$ (e.g. 4.55 mm) of the section 28 of the blood return line 7;
- calculating an initial section pressure drop coefficient $k_{ret\_line}^{init}$ of the blood return line 7 through the following equation (the same as Eq.5; indeed when initiating blood circulation with a new disposable set, it is safe to assume that no clot is present in the blood return line; in this situation the initial section pressure drop coefficient is provided by Eq. 5):

$$\mathrm{Eq.8)} \qquad k_{ret\_line}^{init} = (128 \times L_{ret}) / (\pi \times d_{ret}^{4})$$

(e.g. 0.031 mmHg/ (ml/min) / (mPa s) or 4.133 Pa/(ml/min)/(mPa s))
- calculating $\Delta P_{ret\_line}^{init}$ - $k_{ret\_line}^{init} \times Qb \times \mu$ (e.g. 14 mmHg or 1866 Pa);
- calculating $P_{ret\_disc}^{init} = P_{Hret\_patient} + \Delta P_{ret\_line}^{init}$ (e.g. 0 mmHg or 0 Pa);
- calculating $P_{ret\_thresh}^{init} = P_{ret\_disc}^{init} + \Delta P_{ret\_safety}$ (e.g. +10 mmHg or +1333.22 pa);
- setting the initial section pressure drop coefficient $k_{ret\_line}^{init}$ as design section pressure drop coefficient of the blood return line: $k_{ret\_line}^{init} = k_{ret\_line}^{design}$;
- calculating an initial circuit pressure drop coefficient $k_{ret\_circ}^{init}$ of the blood return line 7 through the following equation:

$$\mathrm{Eq.9)} \qquad k_{ret\_circ}^{init} = (P_{ret}^{init} - P_{Qb0}) / (\mu \times Qb)$$

(e.g. 0.142 mmHg/ (ml/min) / (mPa s) or 1.893 Pa/(ml/min)/(mPa s))
- setting the initial circuit pressure drop coefficient $k_{ret\_circ}^{init}$ as reference circuit pressure drop coefficient $k_{ret\_circ}^{ref}$ of the blood return line 7;
- calculating an initial catheter pressure drop coefficient $k_{ret\_cath}^{init}$ of the blood return line 7 through the following equation:

$$\mathrm{Eq.10)} \qquad k_{ret\_cath}^{init} = k_{ret\_circ}^{init} - k_{ret\_line}^{design}$$

(e.g. 0.111 mmHg/ (ml/min) / (mPa s) or 1.48 Pa/(ml/min)/(mPa s))

- setting the initial catheter pressure drop coefficient $k_{ret\_cath}^{init}$ as reference catheter pressure drop coefficient $k_{ret\_cath}^{ref}$ of the blood return line 7.

The circuit pressure drop coefficient $k_{ret\_circ}$ of the blood return line 7 is the coefficient related to the pressure drop due both to the return section 31 of the vascular access device 400 and to the section 28 of the blood return line 7 from the connector 7a of the blood return line 7 to the return pressure sensor 26. In other embodiments, the initial section pressure drop coefficient of the blood return line $k_{ret\_line}^{init}$ at the start of the extracorporeal blood treatment may also be derived from experimental measurements (measurements (in the scenario where the system may identify the return line configuration, either unique or in relation to the set type).

During the extracorporeal blood treatment, the control unit 100 is configured for performing the following steps:

- measuring the pressure return line $P_{ret}$;
- calculating the circuit pressure drop coefficient $k_{ret\_circ}$ of the blood return line 7 through the following equation:

$$Eq.11) \quad k_{ret\_circ} = (P_{ret} - P_{Qb0}) / (\mu \times Qb)$$

- comparing each new value of the circuit pressure drop coefficient $k_{ret\_circ}$ of the blood return line 7 to the reference circuit pressure drop coefficient $k_{ret\_circ}^{ref}$ of the blood return line 7; and then

if $k_{ret\_circ}$ is greater than $k_{ret\_circ}^{ref}$, then the section pressure drop coefficient $k_{ret\_line}$ of the blood return line 7 is updated as

$$Eq.12) \quad k_{ret\_line} = k_{ret\_circ} - k_{ret\_cath}^{ref}$$

and the return disconnection pressure $P_{ret\_disc}$ is updated accordingly through equations 6, 3 and 7;

if $k_{ret\_circ}$ is less than or equal to $k_{ret\_circ}^{ref}$, then the section pressure drop coefficient $k_{ret\_line}$ of the blood return line 7 remains unchanged and also the return disconnection pressure $P_{ret\_disc}$ remains unchanged while the reference catheter pressure drop coefficient $k_{ret\_cath}^{ref}$ of the blood return line 7 is updated as $k_{ret\_cath}^{ref} = k_{ret\_circ} - k_{ret\_line}^{design}$ and the reference circuit pressure drop coefficient $k_{ret\_circ}^{ref}$ of the blood return line 7 is updated as $k_{ret\_circ}^{ref} = k_{ret\_circ}$.

In the situation where $k_{ret\_circ}$ is less than $k_{ret\_circ}^{ref}$, the circuit pressure drop coefficient $k_{ret\_circ}$ of the blood return line 7 is lower than ever documented since the start of the treatment.

This means that the catheter pressure drop was initially overestimated, expectedly because of some clotting that has resolved over time, assuming that the section pressure drop coefficient $k_{ret\_line}$ of the blood return line 7 cannot decrease below its initial value.

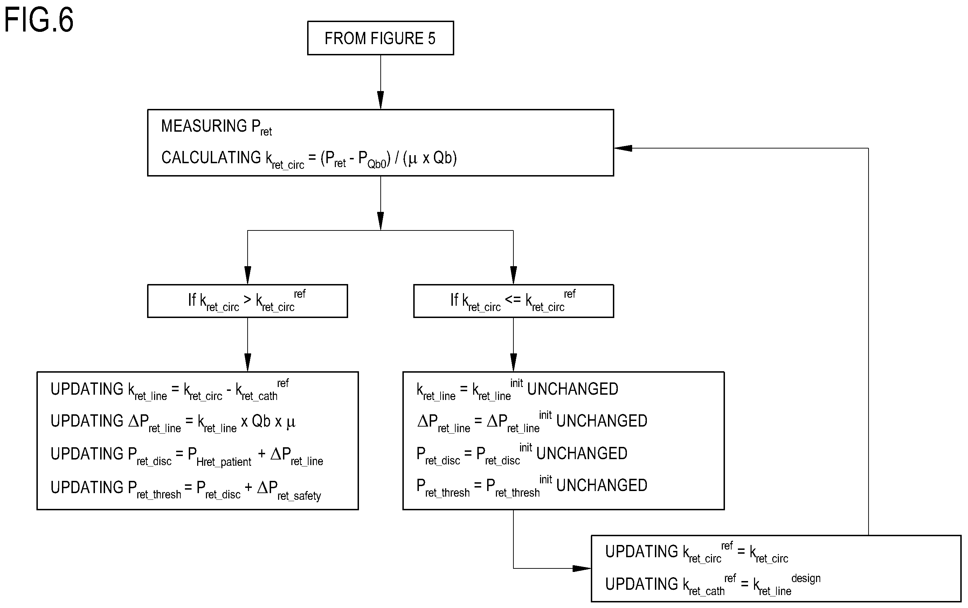

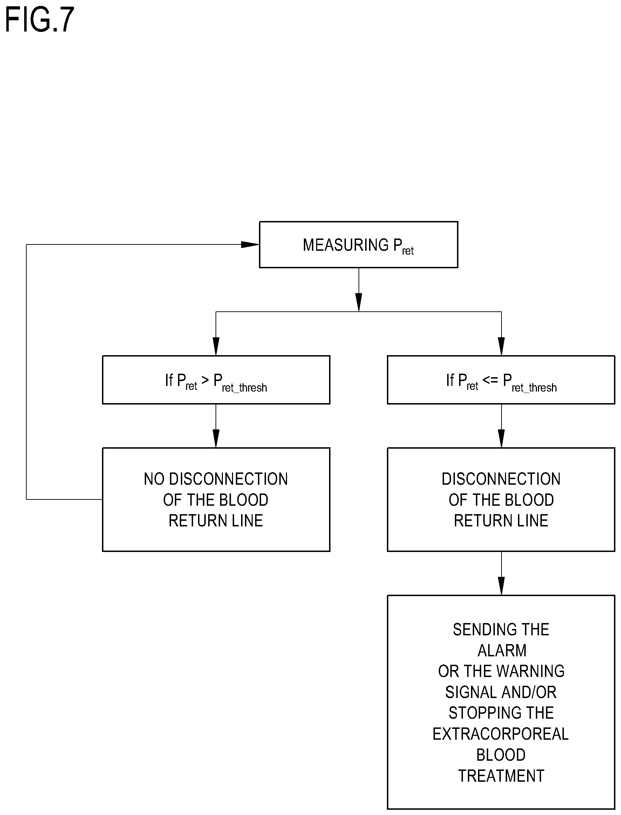

The procedure of Figures 5 and 6 is repeated with a period in the range of minutes, e.g. 1 to 10 minutes, while the control procedure of Figure 7, corresponding to the last part of the method of Example 1 shown in Figure 4, is repeated with a period in the range of seconds or less, e.g. 0.2 to 2 seconds.

The following Table 1 is an example of evolution of the pressure return line $P_{ret}$ over time and related computed pressure drop coefficients and alarm threshold obtained starting from the above illustrative values between brackets of this example 2.

Table 1 (1 mmHg = 133.322 Pa)

| Time | Qb | $P_{ret}$ | $k_{ret\_circ}$ | $k_{ret\_line}$ | $k_{ret\_circ}^{ref}$ | $k_{ret\_cath}^{ref}$ | $P_{ret\_thresh}$ |
|------|------|------|------|------|------|------|------|
| min | ml/min | mmHg | mmHg/ (ml/min) / (mPa s) | | | | mmHg |
| 0 | 150 | 58 | 0.142 | 0.031 | 0.142 | 0.111 | +10 |
| 10 | 150 | 60 | 0.147 | 0.036 | 0.142 | 0.111 | +12 |
| 20 | 150 | 59 | 0.144 | 0.033 | 0.142 | 0.111 | +11 |
| 26[(1)] | 200 | 81 | 0.145 | 0.034 | 0.142 | 0.111 | +30 |
| 30 | 200 | 83 | 0.148 | 0.037 | 0.142 | 0.111 | +32 |
| 40[(2)] | 200 | 96 | 0.170 | 0.059 | 0.142 | 0.111 | +45 |
| 50[(2)] | 200 | 101 | 0.178 | 0.067 | 0.142 | 0.111 | +50 |

(continued)

| Time | Qb | $P_{ret}$ | $k_{ret\_circ}$ | $k_{ret\_line}$ | $k_{ret\_circ}^{ref}$ | $k_{ret\_cath}^{ref}$ | $P_{ret\_thresh}$ |
|---|---|---|---|---|---|---|---|
| min | ml/min | mmHg | mmHg/ (ml/min) / (mPa s) | | | | mmHg |
| 60[2] | 200 | 103 | 0.182 | 0.070 | 0.142 | 0.111 | +52 |
| 70[3] | 200 | 88 | 0.157 | 0.045 | 0.142 | 0.111 | +37 |
| 80[3] | 200 | 82 | 0.147 | 0.039 | 0.142 | 0.111 | +34 |
| 90[3] | 200 | 77 | 0.138 | 0.031 | 0.138 | 0.107 | +29 |
| 100 | 200 | 81 | 0.145 | 0.038 | 0.138 | 0.107 | +33 |
| *(1) blood flow rate change at time 26 min (values a few seconds after the flow change)* *(2) simulation of some clotting in the return circuit* *(3) recovery from clotting* | | | | | | | |

Once the pressure alarm threshold $P_{ret\_thresh}$ has been calculated, the control unit 100 is configured for performing the following steps:

- receiving the measured return pressure $P_{ret}$ and comparing with the pressure alarm threshold $P_{ret\_thresh}$ of the blood return line 7 (with a second or a few seconds frequency);
- sending the alarm or the warning signal and/or stopping the extracorporeal blood treatment if the measured return pressure $P_{ret}$ is equal to or lower than the pressure alarm threshold $P_{ret\_thresh}$.

**Example** 3

Example 3 may be a variant embodiment of Example 1 or of Example 2 wherein the blood viscosity $\mu$ is calculated by the control unit 100.

The control unit 100 is configured for receiving a blood hematocrit Hct and a blood temperature T and for calculating the blood viscosity $\mu$ through the following equation:

$$Eq.13) \quad \mu = e^{(1,8/T)} \times 5,54 \times e^{2,3 \times (Hct/100)}$$

The hematocrit Hct and the blood temperature T may be entered by an operator in the control unit through the interface 110 or may be measured by sensors operatively connected to the control unit 100 and automatically transmitted to the control unit 100. For instance, an optical sensor may be used to measure hematocrit "Htc".

**Disconnection of withdrawal line**

The following equations pertaining to the blood withdrawal line 6 are similar to those related to the return line 7.

$$Eq.14) \quad P_{with} = P_{offset\_with} - \Delta P_{with\_cath} - \Delta P_{with\_line}$$

(see Eq. 1)

$$Eq.15) \quad P_{offset\_with} = P_{with\_Qb0} = P_{venous} + P_{Hwith\_patient}$$

(see Eq. 2)

$$Eq.16) \quad P_{with} = P_{venous} + P_{Hwith\_patient} - \Delta P_{with\_cath} - \Delta P_{with\_line}$$

(see Eq. 1')

$$Eq.17) \quad P_{with\_disc} = P_{Hwith\_patient} - \Delta P_{with\_line} \quad (see\ Eq.\ 3)$$

wherein

$P_{with}$: measured withdrawal pressure from the withdrawal pressure sensor 25;

$P_{with\_Qb0}$: withdrawal pressure at the measurement location when there is no blood flow rate (Qb = 0);

$P_{Hwith\_patient}$ : hydrostatic pressure difference in the blood withdrawal line 6 due to a difference in height between the vascular access device 400 and the withdrawal pressure sensor 25;

$\Delta P_{with\_cath}$: pressure drop in the withdrawal section 30 of the vascular access device 400;

$\Delta P_{with\_line}$: section pressure drop in the blood withdrawal line 6 due to the section 27 of the blood withdrawal line 6 from the connector 6a of the blood withdrawal line 6 to the withdrawal pressure sensor 25;

$P_{with\_disc}$: withdrawal disconnection pressure.

Equation 17 is used to calculate the withdrawal disconnection pressure immediately after a disconnection event.

Differently, since after disconnection of the withdrawal line 6 the blood pump 10 sucks air and the withdrawal line 6 is emptied of blood and filled with air, after an amount of time (e.g. 1 s to 2 s) the withdrawal disconnection pressure $P_{with\_disc}$ may be considered negligible and set to zero ($P_{with\_disc}$ = 0).

**Example 4**

Example 4 is analogous to Example 1. In order to detect the disconnection of the blood withdrawal line 6, before starting the blood treatment, while the blood pump 10, the effluent pump 13, the dialysis pump 15, the pre-blood pump 20, the pre-infusion pump 22 and the post-infusion pump 24 are stopped and the blood flow rate is zero (Qb0), the control unit 100 is configured for performing the following steps:

- measuring the withdrawal pressure $P_{with\_Qb0}$ at the measurement location;
- receiving the patient central venous pressure $P_{venous}$; and
- calculating the hydrostatic pressure difference $P_{Hwith\_patient}$ in the blood withdrawal line 6 starting from equation 15) as:

$$Eq.18) \quad P_{Hwith\_patient} = P_{with\_Qb0} - P_{venous}$$

After starting the blood treatment, the control unit 100 is configured for performing the following steps:

- receiving the blood flow rate Qb;
- receiving the blood viscosity $\mu$ (which may be calculated as disclosed in Example 3);
- receiving a length $L_{with}$ and an internal diameter $d_{with}$ of the section 27 of the blood withdrawal line 6;
- calculating a section pressure drop coefficient of the blood withdrawal line $k_{with\_line}$ through the following equation:

$$Eq.19) \quad k_{with\_line} = (128 \times L_{with})/(\pi \times d_{with}^4)$$

(analogous to Eq. 5)
- calculating the pressure drop $\Delta P_{With\_line}$ in the section 27 of the blood withdrawal line 6 through the following equation:

$$Eq.20) \quad \Delta P_{with\_line} = k_{with\_line} \times Qb \times \mu$$

(analogous to Eq. 6)
- calculating the withdrawal disconnection pressure $P_{with\_disc}$ immediately after disconnection through equation 17;
- setting a pressure alarm threshold $P_{with\_thresh}$ of the blood withdrawal line 6 as:

$$Eq.21) \quad P_{with\_thresh} = P_{with\_disc} - \Delta P_{with\_safety}$$

(safety margin)
- receiving the measured return pressure $P_{with}$ and comparing with the pressure alarm threshold $P_{with\_thresh}$ of the blood withdrawal line 6;
- sending the alarm or the warning signal and/or stopping the extracorporeal blood treatment if the measured withdrawal pressure $P_{with}$ is equal to or greater than the pressure alarm threshold $P_{with\_thresh}$.

If $P_{with} < P_{with\_thresh}$ no disconnection of the blood withdrawal line 6;

If $P_{with} >= P_{with\_thresh}$ disconnection of the blood withdrawal line 6.

**Example 5**

Example 5 is analogous to Example 2. According to Example 5, the section pressure drop coefficient $k_{with\_line}$ of the blood withdrawal line 6 and consequently also the pressure drop $\Delta P_{with\_line}$ in the section 27 of the blood withdrawal line 6, the withdrawal disconnection pressure $P_{with\_disc}$ and the pressure alarm threshold $P_{with\_thresh}$ are continuously updated during the extracorporeal blood treatment.

At the start of the extracorporeal blood treatment, an initial section pressure drop coefficient $k_{with\_line}^{init}$ of the blood withdrawal line 6 is calculated through the following equation (same as Eq.19):

$$Eq.22) \quad k_{with\_line}^{init} = (128 \times L_{with}) / (\pi \times d_{with}^{4})$$

The initial section pressure drop coefficient $k_{with\_line}^{init}$ is set as design section pressure drop coefficient $k_{with\_line}^{design}$ of the blood withdrawal line 6.

An initial circuit pressure drop coefficient $k_{with\_circ}^{init}$ of the blood withdrawal line 7 is calculated through the following equation:

$$Eq.23) \quad k_{with\_circ}^{init} = (P_{Qb0} - P_{with}^{init}) / (\mu \times Qb)$$

(analogous to Eq. 9)

The initial circuit pressure drop coefficient $k_{with\_circ}^{init}$ is set as reference circuit pressure drop coefficient $k_{with\_circ}^{ref}$ of the blood withdrawal line 6.

An initial catheter pressure drop coefficient $k_{with\_cath}^{init}$ of the blood withdrawal line 6 through the following equation:

$$Eq.24) \quad k_{with\_cath}^{init} = k_{with\_circ}^{init} - k_{with\_line}^{design}$$

(analogous to Eq. 10)

The initial catheter pressure drop coefficient $k_{with\_cath}^{init}$ is set as reference catheter pressure drop coefficient $k_{with\_cath}^{ref}$ of the blood withdrawal line 6.

During the extracorporeal blood treatment, the control unit 100 measures the pressure withdrawal line $P_{with}$ and calculates the circuit pressure drop coefficient $k_{with\_circ}$ of the blood withdrawal line 6 through the following equation:

$$Eq.25) \quad k_{with\_circ} = (P_{Qb0} - P_{with}) / (\mu \times Qb)$$

(analogous to Eq. 11)

Each new value of the circuit pressure drop coefficient $k_{with\_circ}$ of the blood withdrawal line 6 is compared to the reference circuit pressure drop coefficient $k_{with\_circ}^{ref}$ of the blood withdrawal line 6; and then:

if $k_{with\_circ}$ is greater than $k_{with\_circ}^{ref}$, then the section pressure drop coefficient $k_{with\_line}$ of the blood withdrawal line 6 is updated as

$$Eq.26) \quad k_{with\_line} = k_{with\_circ} - k_{with\_cath}^{ref}$$

(analogous to Eq. 12) and the withdrawal disconnection pressure $P_{with\_disc}$ is updated accordingly through equations 20, 17 and 21;

if $k_{with\_circ}$ is less than $k_{with\_circ}^{ref}$, then the section pressure drop coefficient $k_{with\_line}$ of the blood withdrawal line 6 remains unchanged and also the withdrawal disconnection pressure $P_{with\_disc}$ remains unchanged while the reference catheter pressure drop coefficient $k_{with\_cath}^{ref}$ of the blood withdrawal line 6 is updated as $k_{with\_cath}^{ref} = k_{with\_circ} - k_{with\_line}^{design}$ and the reference circuit pressure drop coefficient $k_{with\_circ}^{ref}$ of the blood withdrawal line 6 is updated as $k_{with\_circ}^{ref} = k_{with\_circ}$.

**Example 6**

According to Examples 2 and 5, a consistency check of the circuit pressure drop coefficient $k_{ret\_circ}$ of the blood return line 7 or of the circuit pressure drop coefficient $k_{with\_circ}$ of the blood withdrawal line 7 may also be performed.

Equations and computation steps at start and during therapy are unchanged.

For instance, referring to Example 2, where the access device 400 (catheter) type is identified and its pressure drop coefficient known ($k_{ret\_cath}^{design}$), the previous algorithm of Example 2 may be slightly tuned. The change consists in an additional check for consistency of the return circuit (or catheter) pressure drop coefficient through the following equation.

$$Eq.27) \qquad k_{ret\_circ} > k_{ret\_cath}^{design} + k_{ret\_line}^{design}$$

In case above equation is not verified - within the accuracy limits of the measurements - this may drive a confirmation of the catheter type as well as suspicion of return pressure malfunction if the same outcome occurs after repetition of the measuring sequence.

While the invention has been described in connection with what is presently considered to be the most practical and preferred embodiment, it is to be understood that the invention is not to be limited to the disclosed embodiment, but on the contrary, is intended to cover modifications included within the scope of the appended claims.

**Claims**

1. Apparatus for extracorporeal blood treatment, comprising:

    a filtration unit (2);
    an extracorporeal blood circuit having a blood withdrawal line (6) connected to an inlet of the filtration unit (2) and a blood return line (7) connected to an outlet of the filtration unit (2), said extracorporeal blood circuit being configured for connection to a cardiovascular system of a patient (P); the extracorporeal blood circuit comprising at least one connector (6a, 7a) for connection to a vascular access device (400) fixed to the patient (P);
    at least one pressure sensor (25, 26) configured to detect a pressure in at least one measurement location in the extracorporeal blood circuit;
    a blood pump (10) configured to control the flow of blood through the extracorporeal blood circuit;
    a control unit (100) connected to the blood pump (10) and to the at least one pressure sensor (25, 26), the control unit (100) being configured for detecting a disconnection between the at least one connector (6a, 7a) of the extracorporeal blood circuit and the vascular access device (400), by:

        - calculating a hydrostatic pressure difference ($P_{H\_patient}$) due to a difference in height between the vascular access device (400) and said at least one pressure sensor (25, 26);
        - calculating a section pressure drop ($\Delta P_{line}$) due to a section of the blood circuit from the connector (6a, 7a) to said at least one pressure sensor (25, 26);
        - calculating a disconnection pressure ($P_{disc}$) as an algebraic sum of the hydrostatic pressure difference ($P_{H\_patient}$) and the section pressure drop ($\Delta P_{line}$);
        - setting a pressure alarm threshold ($P_{thresh}$) as a function of the disconnection pressure ($P_{disc}$);

    wherein, during an extracorporeal blood treatment, the control unit (100) is configured for:

        - receiving a measured pressure (P) from the at least one pressure sensor (25, 26);
        - detecting a disconnection of the least one connector (6a, 7a) from the vascular access device (400) by comparing the measured pressure (P) with the pressure alarm threshold ($P_{thresh}$).

2. The apparatus of claim 1, wherein the disconnection pressure ($P_{disc}$) is a constant along at least part of the extracorporeal blood treatment or wherein the disconnection pressure ($P_{disc}$) is continuously updated along at least part of the extracorporeal blood treatment.

3. The apparatus of claim 1 or 2, wherein the calculation of the hydrostatic pressure difference ($P_{H\_patient}$) is performed before starting the extracorporeal blood treatment and optionally repeated at least one time along the extracorporeal blood treatment.

4. The apparatus of claim 1 or 2 or 3, wherein the calculation of the section pressure drop ($\Delta P_{line}$) is performed before starting the extracorporeal blood treatment and optionally repeated at least one time along the extracorporeal blood treatment or wherein the section pressure drop ($\Delta P_{line}$) is continuously updated along at least part of the extracorporeal blood treatment.

5. The apparatus of any of claims 1 to 4, wherein, in order to calculate the hydrostatic pressure difference ($P_{H\_patient}$), the control unit (100) is configured for:

- stopping the blood pump (10) or keeping the blood pump (10) stopped to obtain a zero a blood flow rate (Qb0);
- receiving a static pressure ($P_{Qb0}$) from the at least one pressure sensor (25, 26) while the blood flow rate is zero (Qb0);
- receiving a patient central venous pressure ($P_{venous}$);
- calculating the hydrostatic pressure difference ($P_{H\_patient}$) as difference between the static return pressure ($P_{Qb0}$) and the central venous pressure ($P_{venous}$).

6. The apparatus of claim 5, wherein the patient central venous pressure ($P_{venous}$) is measured or is set as a default value, optionally between +799.93 Pa and +1599.87 Pa, optionally of +1333.22 Pa.

7. The apparatus of any of claims 1 to 6, wherein, in order to calculate the section pressure drop ($\Delta P_{line}$), the control unit (100) is configured for:

- receiving a blood flow rate (Qb);
- receiving or calculating a blood viscosity ($\mu$);
- receiving or calculating a section pressure drop coefficient ($k_{line}$) function of a geometry of the section of the blood circuit;
- calculating the section pressure drop ($\Delta P_{line}$) as function of the blood flow rate (Qb), the blood viscosity ($\mu$) and said section pressure drop coefficient ($k_{line}$).

8. The apparatus of claim 7, wherein the section pressure drop coefficient ($k_{line}$) is a constant, optionally wherein the section pressure drop coefficient ($k_{line}$) is given by the following equation: $k_{line} = (128 \times L)/(\pi \times d^4)$; where L is a length of the section and d is an internal diameter of the section.

9. The apparatus of claim 7, wherein the control unit (100) is configured for updating the section pressure drop coefficient ($k_{line}$) and consequently also the disconnection pressure ($P_{disc}$) during the extracorporeal blood treatment.

10. The apparatus of claim 9 when claim 7 depends on claim 5, wherein an initial section pressure drop coefficient ($k_{line}^{init}$) at the start of the extracorporeal blood treatment is given, optionally by the following equation: $k_{line}^{init} = (128 \times L)/(\pi \times d^4)$, and it is set as design section pressure drop coefficient ($k_{line}^{design}$); where L is a length of the section and d is an internal diameter of the section; wherein, in order to update the section pressure drop coefficient ($k_{line}$) during the extracorporeal blood treatment, the control unit (100) is configured for:

- calculating an initial circuit pressure drop coefficient ($k_{circ}^{init}$) as $k_{circ}^{init} = | (P^{init} - P_{Qb0})| / (\mu \times Qb)$ and set it as reference circuit pressure drop coefficient ($k_{circ}^{ref}$);
- calculating an initial catheter pressure drop coefficient ($k_{cath}^{init}$) as $k_{cath}^{init} = k_{circ}^{irit} - k_{line}^{design}$ and set it as reference catheter pressure drop coefficient ($k_{cath}^{ret}$);
- during the extracorporeal blood treatment, measuring the pressure (P) and calculating the circuit pressure drop coefficient as $k_{circ} = (P - P_{Qb0}) / (\mu \times Qb)$;
- comparing each new value of the circuit pressure drop coefficient ($k_{circ}$) to the reference circuit pressure drop coefficient ($k_{circ}^{ref}$) and:

  if $k_{circ}$ is greater than $k_{circ}^{ref}$ then the section pressure drop coefficient ($k_{line}$) is updated as $k_{line} = k_{circ} - k_{cath}^{ref}$ and the disconnection pressure ($P_{disc}$) is updated accordingly;
  if $k_{circ}$ is less than $k_{circ}^{ref}$ then the section pressure drop coefficient ($k_{line}$) remains unchanged and the disconnection pressure ($P_{disc}$) remains unchanged; the reference catheter pressure drop coefficient ($k_{cath}^{ref}$) is updated as $k_{cath}^{ref} = k_{circ} - k_{line}^{design}$; the reference circuit pressure drop coefficient ($k_{circ}^{ref}$) is updated as $k_{circ}^{ref} = k_{circ}$.

11. The apparatus of any of claims 7 to 10, wherein the control unit (100) is configured for:

- receiving a blood hematocrit (Hct);
- optionally receiving a blood temperature (T);

- optionally receiving a protein or albumin concentration (Cp);
- calculating the blood viscosity ($\mu$) from the blood hematocrit (Hct) and optionally the blood temperature (T) and/or protein or albumin concentration (Cp).

12. The apparatus of any of claims 1 to 11, wherein setting the pressure alarm threshold ($P_{thresh}$) comprises:

- setting the pressure alarm threshold ($P_{thresh}$) equal to the disconnection pressure ($P_{disc}$); or
- setting the pressure alarm threshold ($P_{thresh}$) equal to the disconnection pressure ($P_{disc}$) plus a safety margin ($\Delta P_{safety}$);

wherein the control unit (100) is configured for sending an alarm or a warning signal and/or stopping the extracorporeal blood treatment if the measured pressure (P) is outside a pressure range delimited by the pressure alarm threshold ($P_{thresh}$).

13. The apparatus of any of claims 1 to 12, wherein the at least one connector (6a, 7a) comprises a connector (7a) of the blood return line (7), the at least one pressure sensor (25, 26) comprises a return pressure sensor (26) configured to detect a pressure at a measurement location in the blood return line (7) and the control unit (100) is configured for detecting a disconnection between the connector (7a) of the blood return line (7) and the vascular access device (400);

wherein the hydrostatic pressure difference ($P_{H\_patient}$) is a hydrostatic pressure difference ($P_{Hret\_patient}$) in the blood return line (7) due to a difference in height ($H_{ret}$) between the vascular access device (400) and the return pressure sensor (26);
wherein the section pressure drop ($\Delta P_{line}$) is a section pressure drop ($\Delta P_{ret\_line}$) in the blood return line (7) due to a section of the blood return line (7) from the connector (7a) of the blood return line (7) to the return pressure sensor (26);
wherein the disconnection pressure ($P_{disc}$) is a return disconnection pressure ($P_{ret\_disc}$) and is a sum of the hydrostatic pressure difference ($P_{Hret\_patient}$) in the blood return line (7) and the section pressure drop ($\Delta P_{ret\_line}$) in the blood return line (7);
wherein the pressure alarm threshold ($P_{thresh}$) is a pressure alarm threshold ($P_{ret\_thresh}$) of the blood return line (7) and is set as a function of the return disconnection pressure ($P_{ret\_disc}$);
wherein the measured pressure (P) is a measured return pressure ($P_{ret}$) from the return pressure sensor (26);
wherein the disconnection between the connector (7a) of the blood return line (7) and the vascular access device (400) is detected by comparing the measured return pressure ($P_{ret}$) with the pressure alarm threshold ($P_{ret\_thresh}$) of the blood return line (7).

14. The apparatus of claim 13 when depending on claim 12, wherein the control unit (100) is configured for sending the alarm or the warning signal and/or stopping the extracorporeal blood treatment if the measured return pressure ($P_{ret}$) is equal to or lower than the pressure alarm threshold ($P_{ret\_thresh}$).

15. The apparatus of any of claims 1 to 13, wherein the at least one connector (6a, 7a) comprises a connector (6a) of the blood withdrawal line (6), the at least one pressure sensor (25, 26) comprises a withdrawal pressure sensor (25) configured to detect a pressure at a measurement location in the blood withdrawal line (6) and the control unit (100) is configured for detecting a disconnection between the connector (6a) of the blood withdrawal line (6) and the vascular access device (400); wherein the hydrostatic pressure difference ($P_{H\_patient}$) is a hydrostatic pressure difference ($P_{Hwith\_patient}$) in the blood withdrawal line (6) due to a difference in height ($H_{with}$) between the vascular access device (400) and the withdrawal pressure sensor (25);

wherein the section pressure drop ($\Delta P_{line}$) is a section pressure drop ($\Delta P_{with\_line}$) in the blood withdrawal line (6) due to a section of the blood withdrawal line (6) from the connector (6a) of the blood withdrawal line (6) to the withdrawal pressure sensor (25);
wherein the disconnection pressure ($P_{disc}$) is a withdrawal disconnection pressure ($P_{with\_disc}$) and is a difference between the hydrostatic pressure difference ($P_{Hwith\_patient}$) in the blood withdrawal line (6) and the section pressure drop ($\Delta P_{with\_line}$) in the blood withdrawal line (6);
wherein the pressure alarm threshold ($P_{thresh}$) is a pressure alarm threshold ($P_{ret\_thresh}$) of the blood withdrawal line (6) and is set as a function of the withdrawal disconnection pressure ($P_{with\_disc}$);
wherein the measured pressure (P) is a measured withdrawal pressure ($P_{with}$) from the withdrawal pressure sensor (25);
wherein the disconnection between the connector (6a) of the blood withdrawal line (6) and the vascular access device (400) is detected by comparing the measured withdrawal pressure ($P_{with}$) with the pressure alarm threshold ($P_{with\_thresh}$) of the blood withdrawal line (6).

**Patentansprüche**

1. Vorrichtung zur extrakorporalen Blutbehandlung, umfassend: eine Filtrationseinheit (2);

   einen extrakorporalen Blutkreislauf mit einer Blutentnahmeleitung (6), die mit einem Einlass der Filtrationseinheit (2) verbunden ist, und einer Blutrückführleitung (7), die mit einem Auslass der Filtrationseinheit (2) verbunden ist, wobei der extrakorporale Blutkreislauf zum Anschluss an ein kardiovaskuläres System eines Patienten (P) gestaltet ist; wobei der extrakorporale Blutkreislauf wenigstens einen Verbinder (6a, 7a) zum Verbinden mit einer an dem Patienten (P) angebrachten Gefäßzugangsvorrichtung (400) umfasst;
   wenigstens einen Drucksensor (25, 26), gestaltet zum Erfassen eines Drucks an wenigstens einem Messort in dem extrakorporalen Blutkreislauf;
   eine Blutpumpe (10), gestaltet zum Steuern des Flusses von Blut durch den extrakorporalen Blutkreislauf;
   eine Steuereinheit (100), die mit der Blutpumpe (10) und mit dem wenigstens einen Drucksensor (25, 26) verbunden ist, wobei die Steuereinheit (100) dafür gestaltet ist, eine Trennung zwischen dem wenigstens einen Verbinder (6a, 7a) des extrakorporalen Blutkreislaufs und der Gefäßzugangsvorrichtung (400) zu erfassen durch:

   - Berechnen einer hydrostatischen Druckdifferenz ($P_{H\_Patient}$) bedingt durch eine Höhendifferenz zwischen der Gefäßzugangsvorrichtung (400) und dem wenigstens einen Drucksensor (25, 26);
   - Berechnen eines Abschnittsdruckabfalls ($\Delta P_{Leitung}$) bedingt durch einen Abschnitt des Blutkreislaufs von dem Verbinder (6a, 7a) zu dem wenigstens einen Drucksensor (25, 26);
   - Berechnen eines Trennungsdrucks ($P_{Trenn}$) als algebraische Summe der hydrostatischen Druckdifferenz ($P_{H\_Patient}$) und des Abschnittsdruckabfalls ($\Delta P_{Leitung}$);
   - Einstellen eines Druckalarm-Schwellenwerts ($P_{Schwelle}$) als Funktion des Trennungsdrucks ($P_{Trenn}$);

   wobei die Steuereinheit (100) während einer extrakorporalen Blutbehandlung gestaltet ist zum:

   - Empfangen eines gemessenen Drucks (P) von dem wenigstens einen Drucksensor (25, 26);
   - Erkennen einer Trennung des wenigstens einen Verbinders (6a, 7a) von der Gefäßzugangsvorrichtung (400) durch Vergleichen des gemessenen Drucks (P) mit dem Druckalarm-Schwellenwert ($P_{Schwelle}$).

2. Vorrichtung nach Anspruch 1, wobei der Trennungsdruck ($P_{Trenn}$) während wenigstens eines Teils der extrakorporalen Blutbehandlung eine Konstante ist oder wobei der Trennungsdruck ($P_{Trenn}$) während wenigstens eines Teils der extrakorporalen Blutbehandlung kontinuierlich aktualisiert wird.

3. Vorrichtung nach Anspruch 1 oder 2, wobei die Berechnung der hydrostatischen Druckdifferenz ($P_{H\_Patient}$) vor Starten der extrakorporalen Blutbehandlung durchgeführt wird und gegebenenfalls wenigstens einmal während der extrakorporalen Blutbehandlung wiederholt wird.

4. Vorrichtung nach Anspruch 1 oder 2 oder 3, wobei die Berechnung des Abschnittsdruckabfalls ($\Delta P_{Leitung}$) vor Starten der extrakorporalen Blutbehandlung durchgeführt wird und gegebenenfalls wenigstens einmal während der extrakorporalen Blutbehandlung wiederholt wird, oder wobei der Abschnittsdruckabfall ($\Delta P_{Leitung}$) während wenigstens eines Teils der extrakorporalen Blutbehandlung kontinuierlich aktualisiert wird.

5. Vorrichtung nach einem der Ansprüche 1 bis 4, wobei zum Berechnen der hydrostatischen Druckdifferenz ($P_{H\_Patient}$) / die Steuereinheit (100) gestaltet ist zum:

   - Stoppen der Blutpumpe (10) oder gestoppt Halten der Blutpumpe (10), um eine Blutflussrate von null (Qb0) zu erhalten;
   - Empfangen eines statischen Drucks ($P_{Qb0}$) von dem wenigstens einen Drucksensor (25, 26), während die Blutflussrate null ist (Qb0);
   - Empfangen eines zentralen Venendrucks ($P_{Vene}$) des Patienten;
   - Berechnen der hydrostatischen Druckdifferenz ($P_{H\_Patient}$) als Differenz zwischen dem statischen Rücklaufdruck ($P_{Qb0}$) und dem zentralen Venendruck ($P_{Vene}$).

**6.** Vorrichtung nach Anspruch 5, wobei der zentrale Venendruck ($P_{Vene}$) des Patienten gemessen wird oder als ein Standardwert eingestellt wird, gegebenenfalls zwischen +799,93 Pa und +1599,87 Pa, gegebenenfalls +1333,22 Pa.

**7.** Vorrichtung nach einem der Ansprüche 1 bis 6, wobei die Steuereinheit (100) zum Berechnen des Abschnittsdruckabfalls ($\Delta P_{Leitung}$) gestaltet ist zum:

- Empfangen einer Blutflussrate (Qb);
- Empfangen oder Berechnen einer Blutviskosität ($\mu$);
- Empfangen oder Berechnen einer Abschnittsdruckabfallkoeffizient ($k_{Leitung}$) als Funktion der Geometrie des Abschnitts des Blutkreislaufs;
- Berechnen des Abschnittsdruckabfalls ($\Delta P_{Leitung}$) als Funktion der Blutflussrate (Qb), der Blutviskosität ($\mu$) und des Abschnittsdruckabfallkoeffizienten ($k_{Leitung}$).

**8.** Vorrichtung nach Anspruch 7, wobei der Abschnittsdruckabfallkoeffizient ($k_{Leitung}$) eine Konstante ist, wobei gegebenenfalls der Abschnittsdruckabfallkoeffizient ($k_{Leitung}$) durch die folgende Gleichung gegeben ist: $k_{Leitung} = (128 \times L) / (\pi \times d^4)$; wobei L eine Länge des Abschnitts ist und d ein Innendurchmesser des Abschnitts ist.

**9.** Vorrichtung nach Anspruch 7, wobei die Steuereinheit (100) dafür gestaltet ist, den Abschnittsdruckabfallkoeffizienten ($k_{Leitung}$) und damit auch den Trenndruck ($P_{Trenn}$) während der extrakorporalen Blutbehandlung zu aktualisieren.

**10.** Vorrichtung nach Anspruch 9, wenn Anspruch 7 von Anspruch 5 abhängt, wobei ein Anfangs-Abschnittsdruckabfallkoeffizient ($k_{Leitung}^{Anfang}$) zu Beginn der extrakorporalen Blutbehandlung gegeben ist, gegebenenfalls durch die folgende Gleichung: $k_{Leitung}^{Anfang} - (128 \times L) / (\pi \times d^4)$, und als bauartbedingter Abschnittsdruckabfallkoeffizient ($k_{Leitung}^{Bauart}$) eingestellt ist; wobei L eine Länge des Abschnitts ist und d ein Innendurchmesser des Abschnitts ist; wobei die Steuereinheit (100) zum Aktualisieren des Abschnittsdruckabfallkoeffizienten ($k_{Leitung}$) während der extrakorporalen Blutbehandlung gestaltet ist zum:

- Berechnen eines Anfangs-Kreislaufdruckabfallkoeffizienten ($k_{Kreis}^{Anfang}$) als $k_{Kreis}^{Anfang} = | (p^{Anfang} - P_{Qb0}) | / (\mu \times Qb)$ und Einstellen davon als Referenz-Kreislaufdruckabfallkoeffizient ($k_{Kreis}^{Ref}$);
- Berechnen eines Anfangs-Katheterdruckabfallkoeffizienten ($k_{Kath}^{Anfang}$) als $k_{Kath}^{Anfang} = k_{Kreis}^{Anfang} - k_{Leitung}^{Bauart}$ und Festlegen davon als Referenz-Katheterdruckabfallkoeffizient ($k_{Kath}^{Ref}$);
- während der extrakorporalen Blutbehandlung Messen des Drucks (P) und Berechnen des Kreislaufdruckabfallkoeffizienten als $k_{Kreis} = (P - P_{Qb0}) / (\mu \times Qb)$;
- Vergleichen jedes neuen Werts des Kreislaufdruckabfallkoeffizienten ($k_{Kreis}$) mit dem Referenz-Kreislaufdruckabfallkoeffizienten ($k_{Kreis}^{Ref}$) und:

    wenn $k_{Kreis}$ größer ist als $k_{Kreis}^{Ref}$, Aktualisieren des Abschnittsdruckabfallkoeffizienten ($k_{Leitung}$) als $k_{Leitung} = k_{Kreis} - k_{Kath}^{Ref}$ und entsprechendes Aktualisieren des Trennungsdrucks ($P_{Trenn}$);
    wenn $k_{Kreis}$ kleiner ist als $k_{Kreis}^{Ref}$, unverändert lassen des Abschnittsdruckabfallkoeffizienten ($k_{Leitung}$) und des Trennungsdrucks ($P_{Trenn}$); Aktualisieren des Referenz-Katheterdruckabfallkoeffizienten ($k_{Kath}^{Ref}$) als $k_{Kath}^{Ref} = k_{Kreis}s - k_{Leitung}^{Bauart}$; Aktualisieren des Referenz-Kreislaufdruckabfallkoeffizienten ($k_{Kreis}^{Ref}$) als $k_{Kreis}^{Ref} = k_{Kreis}$.

**11.** Vorrichtung nach einem der Ansprüche 7 bis 10, wobei die Steuereinheit (100) gestaltet ist zum:

- Erhalten eines Bluthämatokrits (Hct);
- gegebenenfalls Erhalten einer Bluttemperatur (T);
- gegebenenfalls Erhalten einer Protein- oder Albuminkonzentration (Cp);
- Berechnen der Blutviskosität ($\mu$) aus dem Bluthämatokrit (Hct) und gegebenenfalls der Bluttemperatur (T) und/oder Protein- oder Albuminkonzentration (Cp).

**12.** Vorrichtung nach einem der Ansprüche 1 bis 11, wobei Einstellen des Druckalarm-Schwellenwerts ($P_{Schwelle}$) umfasst:

- Einstellen des Druckalarm-Schwellenwerts ($P_{Schwelle}$) auf den Trennungsdruck ($P_{Trenn}$); oder
- Einstellen des Druckalarm-Schwellenwerts ($P_{Schwelle}$) auf den Trennungsdruck ($P_{Trenn}$) plus einer Sicherheitsspanne ($\Delta P_{Sicherheit}$);

wobei die Steuereinheit (100) zum Ausgeben eines Alarms oder eines Warnsignals und/oder Stoppen der extrakorporalen Blutbehandlung gestaltet ist, wenn der gemessene Druck (P) außerhalb eines Druckbereichs liegt, der von dem Druckalarm-Schwellenwert ($P_{Schwelle}$) begrenzt ist.

13. Vorrichtung nach einem der Ansprüche 1 bis 12, wobei der wenigstens eine Verbinder (6a, 7a) einen Verbinder (7a) der Blutrückführleitung (7) umfasst, wobei der wenigstens eine Drucksensor (25, 26) einen Rücklaufdrucksensor (26) umfasst, der dafür gestaltet ist, einen Druck an einer Messstelle in der Blutrückführleitung (7) zu erfassen, und die Steuereinheit (100) dafür gestaltet ist, eine Trennung zwischen dem Verbinder (7a) der Blutrückführleitung (7) und der Gefäßzugangsvorrichtung (400) zu erfassen;

wobei die hydrostatische Druckdifferenz ($P_{H\_Patient}$) eine hydrostatische Druckdifferenz ($P_{Hrück\_Patient}$) in der Blutrückführleitung (7) bedingt durch eine Höhendifferenz ($H_{Rück}$) zwischen der Gefäßzugangsvorrichtung (400) und dem Rücklaufdrucksensor (26) ist;
wobei der Abschnittsdruckabfall ($\Delta P_{Leitung}$) ein Abschnittsdruckabfall ($\Delta P_{Rück\_Leitung}$) in der Blutrückführleitung (7) bedingt durch einen Abschnitt der Blutrückführleitung (7) von dem Verbinder (7a) der Blutrückführleitung (7) zu dem Rücklaufdrucksensor (26) ist;
wobei der Trennungsdruck ($P_{Trenn}$) ein Rücklauftrennungsdruck ($P_{Rück\_Kreis}$) ist und eine Summe aus der hydrostatischen Druckdifferenz ($P_{HRück\_Patient}$) in der Blutrückführleitung (7) und dem Abschnittsdruckabfall ($\Delta P_{Rück\_Leitung}$) in der Blutrückführleitung (7) ist;
wobei der Druckalarm-Schwellenwert ($P_{Schwelle}$) ein Druckalarm-Schwellenwert ($P_{Rück\_Schwelle}$) der Blutrückführleitung (7) ist und als Funktion des Rücklauftrennungsdruckr ($P_{Rück\_Kreis}$) eingestellt wird;
wobei der gemessene Druck (P) ein gemessener Rücklaufdruck ($P_{Rück}$) von dem Rücklaufdrucksensor (26) ist;
wobei die Trennung zwischen dem Verbinder (7a) der Blutrückführleitung (7) und der Gefäßzugangsvorrichtung (400) durch Vergleichen des gemessenen Rücklaufdrucks ($P_{Rück}$) mit dem Druckalarm-Schwellenwert ($P_{Rück\_Schwelle}$) der Blutrückführleitung (7) erfasst wird.

14. Vorrichtung nach Anspruch 13, wenn abhängig von Anspruch 12, wobei die Steuereinheit (100) zum Ausgeben des Alarms oder des Warnsignals und/oder Stoppen der extrakorporalen Blutbehandlung gestaltet ist, wenn der gemessene Rücklaufdruck ($P_{Rück}$) gleich oder niedriger als die Druckalarm-Schwellenwert ($P_{Rück\_Schwelle}$) ist.

15. Vorrichtung nach einem der Ansprüche 1 bis 13, wobei der wenigstens eine Verbinder (6a, 7a) einen Verbinder (6a) der Blutentnahmeleitung (6) umfasst, wobei der wenigstens eine Drucksensor (25, 26) einen Entnahmedrucksensor (25) umfasst, der dafür gestaltet ist, einen Druck an einer Messstelle in der Blutentnahmeleitung (6) zu erfassen, und die Steuereinheit (100) dafür gestaltet ist, eine Trennung zwischen dem Verbinder (6a) der Blutentnahmeleitung (6) und der Gefäßzugangsvorrichtung (400) zu erfassen;

wobei die hydrostatische Druckdifferenz ($P_{H\_Patient}$) eine hydrostatische Druckdifferenz ($P_{nenen\_Patient}$) in der Blutentnahmeleitung (6) bedingt durch eine Höhendifferenz ($H_{Entn}$) zwischen der Gefäßzugangsvorrichtung (400) und dem Entnahmedrucksensor (25) ist;
wobei der Abschnittsdruckabfall ($\Delta P_{Leitung}$) ein Abschnittsdruckabfall ($\Delta P_{Entn\_Leitung}$) in der Blutentnahmeleitung (6) bedingt durch einen Abschnitt der Blutentnahmeleitung (6) von dem Verbinder (6a) der Blutentnahmeleitung (6) zu dem Entnahmedrucksensor (25) ist;
wobei der Trenndruck ($P_{Kreis}$) ein Entnahmetrenndruck ($P_{Ent\_Kreis}$) ist und eine Differenz zwischen der hydrostatischen Druckdifferenz ($P_{HEntn\_Patient}$) in der Blutentnahmeleitung (6) und dem Abschnittsdruckabfall ($\Delta P_{Entn\_Leitung}$) in der Blutentnahmeleitung (6) ist;
wobei die Druckalarm-Schwellenwert ($P_{Schwelle}$) ein Druckalarm-Schwellenwert ($P_{Rück\_Schwelle}$) der Blutentnahmeleitung (6) ist und als Funktion des Entnahmetrennungsdrucks ($P_{Ent\_Kreis}$) eingestellt wird; wobei der gemessene Druck (P) ein gemessener Entnahmedruck ($P_{Entn}$) von dem Entnahmedrucksensor (25) ist;
wobei die Trennung zwischen dem Verbinder (6a) der Blutentnahmeleitung (6) und der Gefäßzugangsvorrichtung (400) durch Vergleichen des gemessenen Entnahmedrucks ($P_{Entn}$) mit dem Druckalarm-Schwellenwert ($P_{Entn\_Schwelle}$) der Blutentnahmeleitung (6) erfasst wird.

**Revendications**

1. Appareil de traitement extracorporel du sang, comprenant :

une unité de filtration (2) ;

un circuit extracorporel du sang comportant une ligne de prélèvement de sang (6) reliée à une entrée de l'unité de filtration (2) et une ligne de retour de sang (7) reliée à une sortie de l'unité de filtration (2), ledit circuit extracorporel du sang étant configuré pour une liaison à un système cardiovasculaire d'un patient (P) ; le circuit extracorporel du sang comprenant au moins un raccord (6a, 7a) pour une liaison à un dispositif d'accès vasculaire (400) fixé au patient (P) ;

au moins un capteur de pression (25, 26) configuré pour détecter une pression dans au moins un emplacement de mesure dans le circuit extracorporel du sang ;

une pompe à sang (10) configurée pour réguler l'écoulement de sang à travers le circuit extracorporel du sang ;

une unité de commande (100) reliée à la pompe à sang (10) et à l'au moins un capteur de pression (25, 26), l'unité de commande (100) étant configurée pour détecter une désolidarisation entre l'au moins un raccord (6a, 7a) du circuit extracorporel du sang et le dispositif d'accès vasculaire (400), par :

- calcul d'une différence de pression hydrostatique ($P_{H\_patient}$) due à une différence de hauteur entre le dispositif d'accès vasculaire (400) et ledit au moins un capteur de pression (25, 26) ;
- calcul d'une chute de pression de section ($\Delta P_{line}$) due à une section du circuit extracorporel du sang du raccord (6a, 7a) audit au moins un capteur de pression (25, 26) ;
- calcul d'une pression de désolidarisation ($P_{disc}$) en tant que somme algébrique de la différence de pression hydrostatique ($P_{H\_patient}$) et de la chute de pression de section ($\Delta P_{line}$) ;
- réglage d'un seuil d'alarme de pression ($P_{thresh}$) en fonction de la pression de désolidarisation ($P_{disc}$) ;

dans lequel, pendant un traitement extracorporel du sang, l'unité de commande (100) est configurée pour :

- recevoir une pression mesurée (P) provenant de l'au moins un capteur de pression (25, 26) ;
- détecter une désolidarisation de l'au moins un raccord (6a, 7a) du dispositif d'accès vasculaire (400) par comparaison de la pression mesurée (P) avec le seuil d'alarme de pression ($P_{thresh}$).

2. Appareil selon la revendication 1, dans lequel la pression de désolidarisation ($P_{disc}$) est une constante le long d'au moins une partie du traitement extracorporel du sang ou dans lequel la pression de désolidarisation ($P_{disc}$) est continuellement mise à jour le long d'au moins une partie du traitement extracorporel du sang.

3. Appareil selon la revendication 1 ou 2, dans lequel le calcul de la différence de pression hydrostatique ($P_{H\_patient}$) est effectué avant le démarrage du traitement extracorporel du sang et éventuellement répété au moins une fois avec le traitement extracorporel du sang.

4. Appareil selon la revendication 1 ou 2 ou 3, dans lequel le calcul de la chute de pression de section ($\Delta P_{line}$) est effectué avant le démarrage du traitement extracorporel du sang et éventuellement répété au moins une fois avec le traitement extracorporel du sang ou dans lequel la chute de pression de section ($\Delta P_{line}$) est continuellement mise à jour avec au moins une partie du traitement extracorporel du sang.

5. Appareil selon l'une quelconque des revendications 1 à 4, dans lequel, afin de calculer la différence de pression hydrostatique ($P_{H\_patient}$), l'unité de commande (100) est configurée pour :

- arrêter la pompe à sang (10) ou maintenir la pompe à sang (10) arrêtée pour obtenir un débit sanguin nul (Qb0) ;
- recevoir une pression statique ($P_{Qb0}$) provenant de l'au moins un capteur de pression (25, 26) pendant que le débit sanguin est nul (Qb0) ;
- recevoir une pression veineuse centrale ($P_{venous}$) du patient ;
- calculer la différence de pression hydrostatique ($P_{H\_patient}$) en tant que différence entre la pression statique de retour ($P_{Qb0}$) et la pression veineuse centrale ($P_{venous}$).

6. Appareil selon la revendication 5, dans lequel la pression veineuse centrale ($P_{venous}$) du patient est mesurée ou est réglée en tant que valeur par défaut, facultativement entre +799,93 Pa et +1 599,87 Pa, facultativement de +1 333,22 Pa.

7. Appareil selon l'une quelconque des revendications 1 à 6, dans lequel, dans le but de calculer la chute de pression de section ($\Delta P_{line}$), l'unité de commande (100) est configurée pour :

- recevoir un débit sanguin (Qb) ;
- recevoir ou calculer une viscosité sanguine ($\mu$) ;

- recevoir ou calculer un coefficient de chute de pression de section ($k_{line}$) fonction d'une géométrie de la section du circuit extracorporel du sang ;
- calculer la chute de pression de section ($\Delta P_{line}$) en fonction du débit sanguin (Qb), de la viscosité sanguine ($\mu$) et dudit coefficient de chute de pression de section ($k_{line}$).

8. Appareil selon la revendication 7, dans lequel le coefficient de chute de pression de section ($k_{line}$) est une constante, éventuellement dans lequel le coefficient de chute de pression de section ($k_{line}$) est donné par l'équation suivante : $k_{line} = (128 \times L) / (\pi \times d^4)$ ; où L est une longueur de la section et d est un diamètre interne de la section.

9. Appareil selon la revendication 7, dans lequel l'unité de commande (100) est configurée pour mettre à jour le coefficient de chute de pression de section ($k_{line}$) et par conséquent également la pression de désolidarisation ($P_{disc}$) pendant le traitement extracorporel du sang.

10. Appareil selon la revendication 9 lorsque la revendication 7 dépend de la revendication 5, dans lequel un coefficient de chute de pression de section initial ($k_{line}^{init}$) au début du traitement extracorporel du sang est donné, facultativement par l'équation suivante : $k_{line}^{init} = (128 \times L) / (\pi \times d^4)$, et il est réglé en tant que coefficient de chute de pression de section de conception ($k_{line}^{design}$) ; où L est une longueur de la section et d est un diamètre interne de la section ; dans lequel, dans le but de mettre à jour le coefficient de chute de pression de section ($k_{line}$) pendant le traitement extracorporel du sang, l'unité de commande (100) est configurée pour :

- calculer un coefficient de chute de pression de circuit initial ($k_{circ}^{init}$) en tant que $k_{circ}^{init} = |(P^{init} - P_{Qb0})| / (\mu \times Qb)$ et le régler en tant que coefficient de chute de pression de circuit de référence ($k_{circ}^{ref}$) ;
- calculer un coefficient de chute de pression de cathéter initial ($k_{cath}^{init}$) en tant que $k_{cath}^{init} = k_{circ}^{init} - k_{line}^{design}$ et le régler en tant que coefficient de chute de pression de cathéter de référence ($k_{cath}^{ref}$) ;
- pendant le traitement extracorporel du sang, mesurer la pression (P) et calculer le coefficient de chute de pression de circuit en tant que $k_{circ} = (P - P_{Qb0}) / (\mu \times Qb)$ ;
- comparer chaque nouvelle valeur du coefficient de chute de pression de circuit ($k_{circ}$) au coefficient de chute de pression de circuit de référence ($k_{circ}^{ref}$) et :

  si $k_{circ}$ est supérieur à $k_{circ}^{ref}$, le coefficient de chute de pression de section ($k_{line}$) est mis à jour en tant que $k_{line} = k_{circ} - k_{cath}^{ref}$ et la pression de désolidarisation ($P_{disc}$) est mise à jour en conséquence ;
  si $k_{circ}$ est inférieur à $k_{circ}^{ref}$, le coefficient de chute de pression de section ($k_{line}$) reste inchangé et la pression de désolidarisation ($P_{disc}$) reste inchangée ; le coefficient de chute de pression de cathéter de référence ($k_{cath}^{ref}$) est mis à jour en tant que $k_{cath}^{ref} = k_{circ} - k_{line}^{design}$ ; le coefficient de chute de pression de circuit de référence ($k_{circ}^{ref}$) est mis à jour en tant que $k_{circ}^{ref} = k_{circ}$.

11. Appareil selon l'une quelconque des revendications 7 à 10, dans lequel l'unité de commande (100) est configurée pour :

- recevoir un hématocrite sanguin (Hct) ;
- facultativement recevoir une température sanguine (T) ;
- facultativement recevoir une concentration de protéines ou d'albumine (Cp) ;
- calculer la viscosité sanguine ($\mu$) à partir de l'hématocrite sanguin (Hct) et facultativement de la température sanguine (T) et/ou de la concentration en protéines ou albumine (Cp).

12. Appareil selon l'une quelconque des revendications 1 à 11, dans lequel le réglage du seuil d'alarme de pression ($P_{thresh}$) comprend :

- le réglage du seuil d'alarme de pression ($P_{thresh}$) égal à la pression de désolidarisation ($P_{disc}$) ; ou
- le réglage du seuil d'alarme de pression ($P_{thresh}$) égal à la pression de désolidarisation ($P_{disc}$) plus une marge de sécurité ($\Delta P_{safety}$) ;

dans lequel l'unité de commande (100) est configurée pour envoyer une alarme ou un signal d'avertissement et/ou arrêter le traitement extracorporel du sang si la pression mesurée (P) se situe en dehors d'une plage de pression délimitée par le seuil d'alarme de pression ($P_{thresh}$).

13. Appareil selon l'une quelconque des revendications 1 à 12, dans lequel l'au moins un raccord (6a, 7a) comprend un raccord (7a) de la ligne de retour de sang (7), l'au moins un capteur de pression (25, 26) comprend un capteur de pression de retour (26) configuré pour détecter une pression à un emplacement de mesure dans la ligne de retour de sang (7) et l'unité de commande (100) est configurée pour détecter une désolidarisation entre le raccord (7a) de la ligne de retour de sang (7) et le dispositif d'accès vasculaire (400) ;

dans lequel la différence de pression hydrostatique ($P_{H\_patient}$) est une différence de pression hydrostatique ($P_{Hret\_patient}$) dans la ligne de retour de sang (7) due à une différence de hauteur ($H_{ret}$) entre le dispositif d'accès vasculaire (400) et le capteur de pression de retour (26) ;
dans lequel la chute de pression de section ($\Delta P_{line}$) est une chute de pression de section ($\Delta P_{ret\_line}$) dans la ligne de retour de sang (7) due à une section de la ligne de retour de sang (7) du raccord (7a) de la ligne de retour de sang (7) au capteur de pression de retour (26) ;
dans lequel la pression de désolidarisation ($P_{disc}$) est une pression de désolidarisation de retour ($P_{ret\_disc}$) et est une somme de la différence de pression hydrostatique ($P_{Hret\_patient}$) dans la ligne de retour de sang (7) et de la chute de pression de section ($\Delta P_{ret\_line}$) dans la ligne de retour de sang (7) ;
dans lequel le seuil d'alarme de pression ($P_{thresh}$) est un seuil d'alarme de pression ($P_{ret\_thresh}$) de la ligne de retour de sang (7) et est réglé en fonction de la pression de désolidarisation de retour ($P_{ret\_disc}$) ;
dans lequel la pression mesurée ($P$) est une pression de retour mesurée ($P_{ret}$) provenant du capteur de pression de retour (26) ;
dans lequel la désolidarisation entre le raccord (7a) de la ligne de retour de sang (7) et le dispositif d'accès vasculaire (400) est détectée par comparaison de la pression de retour mesurée ($P_{ret}$) avec le seuil d'alarme de pression ($P_{ret\_thresh}$) de la ligne de retour de sang (7).

14. Appareil selon la revendication 13 lorsqu'elle dépend de la revendication 12, dans lequel l'unité de commande (100) est configurée pour envoyer l'alarme ou le signal d'avertissement et/ou arrêter le traitement extracorporel du sang si la pression de retour mesurée ($P_{ret}$) est égale ou inférieure au seuil d'alarme de pression ($P_{ret\_thresh}$).

15. Appareil selon l'une quelconque des revendications 1 à 13, dans lequel l'au moins un raccord (6a, 7a) comprend un raccord (6a) de la ligne de prélèvement de sang (6), l'au moins un capteur de pression (25, 26) comprend un capteur de pression de prélèvement (25) configuré pour détecter une pression à un emplacement de mesure dans la ligne de prélèvement de sang (6) et l'unité de commande (100) est configurée pour détecter une désolidarisation entre le raccord (6a) de la ligne de prélèvement de sang (6) et le dispositif d'accès vasculaire (400) ;

dans lequel la différence de pression hydrostatique ($P_{H\_patient}$) est une différence de pression hydrostatique ($P_{Hwith\_patient}$) dans la ligne de prélèvement de sang (6) due à une différence de hauteur ($H_{with}$) entre le dispositif d'accès vasculaire (400) et le capteur de pression de prélèvement (25) ;
dans lequel la chute de pression de section ($\Delta P_{line}$) est une chute de pression de section ($\Delta P_{with\_line}$) dans la ligne de prélèvement de sang (6) due à une section de la ligne de prélèvement de sang (6) du raccord (6a) de la ligne de prélèvement de sang (6) au capteur de pression de prélèvement (25) ;
dans lequel la pression de désolidarisation ($P_{disc}$) est une pression de désolidarisation de prélèvement ($P_{with\_disc}$) et est une différence entre la différence de pression hydrostatique ($P_{Hwith\_patient}$) dans la ligne de prélèvement de sang (6) et la chute de pression de section ($\Delta P_{with\_line}$) dans la ligne de prélèvement de sang (6) ;
dans lequel le seuil d'alarme de pression ($P_{thresh}$) est un seuil d'alarme de pression ($P_{ret\_thresh}$) de la ligne de prélèvement de sang (6) et est réglé en fonction de la pression de désolidarisation de prélèvement ($P_{with\_disc}$) ;
dans lequel la pression mesurée ($P$) est une pression de prélèvement mesurée ($P_{with}$) provenant de capteur de pression de prélèvement (25) ;
dans lequel la désolidarisation entre le raccord (6a) de la ligne de prélèvement de sang (6) et le dispositif d'accès vasculaire (400) est détectée par comparaison de la pression de prélèvement mesurée ($P_{with}$) avec le seuil d'alarme de pression ($P_{with\_thresh}$) de la ligne de prélèvement de sang (6).

FIG.1

FIG.2

FIG.3

BEFORE STARTING THE BLOOD TREATMENT:

MEASURING $P_{ret\_Qb0}$

RECEIVING $P_{venous}$

CALCULATING $P_{Hret\_patient} = P_{ret\_Qb0} - P_{venous}$

---

AFTER STARTING THE BLOOD TREATMENT:

RECEIVING $L_{ret}$ AND $d_{ret}$

CALCULATING $k_{ret\_line} = (128 \times L_{ret}) / (\pi \times d_{ret}^{4})$

RECEIVING Qb AND $\mu$

CALCULATING $\Delta P_{ret\_line} = k_{ret\_line} \times Qb \times \mu$

---

CALCULATING $P_{ret\_disc} = P_{Hret\_patient} + \Delta P_{ret\_line}$

CALCULATING $P_{ret\_thresh} = P_{ret\_disc} + \Delta P_{ret\_safety}$

---

MEASURING $P_{ret}$

If $P_{ret} > P_{ret\_thresh}$

NO DISCONNECTION OF THE BLOOD RETURN LINE

If $P_{ret} <= P_{ret\_thresh}$

DISCONNECTION OF THE BLOOD RETURN LINE

SENDING THE ALARM OR THE WARNING SIGNAL AND/OR STOPPING THE EXTRACORPOREAL BLOOD TREATMENT

FIG.4

BEFORE STARTING THE BLOOD TREATMENT:

MEASURING $P_{ret\_Qb0}$

RECEIVING $P_{venous}$

CALCULATING $P_{Hret\_patient} = P_{ret\_Qb0} - P_{venous}$

AFTER STARTING THE BLOOD TREATMENT:

RECEIVING $L_{ret}$ AND $d_{ret}$

CALCULATING $k_{ret\_line}^{init} = (128 \times L_{ret}) / ( \pi \times d_{ret}^{4})$

SETTING $k_{ret\_line}^{init} = k_{ret\_line}^{design}$

RECEIVING Qb AND $\mu$

CALCULATING $\Delta P_{ret\_line}^{init} = k_{ret\_line}^{init} \times Qb \times \mu$

CALCULATING $P_{ret\_disc}^{init} = P_{Hret\_patient} + \Delta P_{ret\_line}^{init}$

CALCULATING $P_{ret\_thresh}^{init} = P_{ret\_disc}^{init} + \Delta P_{ret\_safety}$

CALCULATING $k_{ret\_circ}^{init} = (P_{ret}^{init} - P_{Qb0}) / (\mu \times Qb)$

SETTING $k_{ret\_circ}^{init} = k_{ret\_circ}^{ref}$

CALCULATING $k_{ret\_cath}^{init} = k_{ret\_circ}^{init} - k_{ret\_line}^{design}$

SETTING $k_{ret\_cath}^{init} = k_{ret\_cath}^{ref}$

TO FIGURE 6

FIG.5

# FIG.6

FROM FIGURE 5

MEASURING $P_{ret}$

CALCULATING $k_{ret\_circ} = (P_{ret} - P_{Qb0}) / (\mu \times Qb)$

If $k_{ret\_circ} > k_{ret\_circ}^{ref}$

If $k_{ret\_circ} <= k_{ret\_circ}^{ref}$

UPDATING $k_{ret\_line} = k_{ret\_circ} - k_{ret\_cath}^{ref}$

UPDATING $\Delta P_{ret\_line} = k_{ret\_line} \times Qb \times \mu$

UPDATING $P_{ret\_disc} = P_{Hret\_patient} + \Delta P_{ret\_line}$

UPDATING $P_{ret\_thresh} = P_{ret\_disc} + \Delta P_{ret\_safety}$

$k_{ret\_line} = k_{ret\_line}^{init}$ UNCHANGED

$\Delta P_{ret\_line} = \Delta P_{ret\_line}^{init}$ UNCHANGED

$P_{ret\_disc} = P_{ret\_disc}^{init}$ UNCHANGED

$P_{ret\_thresh} = P_{ret\_thresh}^{init}$ UNCHANGED

UPDATING $k_{ret\_circ}^{ref} = k_{ret\_circ}$

UPDATING $k_{ret\_cath}^{ref} = k_{ret\_line}^{design}$

# FIG.7

```
                            ┌─────────────────────┐
                            │   MEASURING P_ret    │◄──────┐
                            └─────────┬───────────┘        │
                                      │                     │
                  ┌───────────────────┴───────────────────┐ │
                  ▼                                         ▼ │
    ┌──────────────────────────┐          ┌──────────────────────────┐
    │   If P_ret > P_ret_thresh │          │  If P_ret <= P_ret_thresh │
    └────────────┬─────────────┘          └────────────┬─────────────┘
                 ▼                                       ▼
    ┌──────────────────────────┐          ┌──────────────────────────┐
    │     NO DISCONNECTION      │          │      DISCONNECTION        │
    │      OF THE BLOOD         │          │       OF THE BLOOD        │
    │      RETURN LINE          │          │       RETURN LINE         │
    └──────────────────────────┘          └────────────┬─────────────┘
                 │                                       ▼
                 └───────────────────┐     ┌──────────────────────────┐
                                           │      SENDING THE          │
                                           │        ALARM              │
                                           │   OR THE WARNING          │
                                           │   SIGNAL AND/OR           │
                                           │   STOPPING THE            │
                                           │  EXTRACORPOREAL           │
                                           │       BLOOD               │
                                           │     TREATMENT             │
                                           └──────────────────────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018053461 A1
- US 20160354531 A1

- US 20180126062 A1